(19) Europäisches Patentamt
European Patent Office
Office européen des brevets

(11) EP 3 231 426 A1

(12) EUROPEAN PATENT APPLICATION
published in accordance with Art. 153(4) EPC

(43) Date of publication:
**18.10.2017 Bulletin 2017/42**

(21) Application number: **15867977.9**

(22) Date of filing: **08.12.2015**

(51) Int Cl.:
***A61K 31/497*** *(2006.01)* ***A61K 31/496*** *(2006.01)*
***A61P 35/00*** *(2006.01)* ***A61P 43/00*** *(2006.01)*
***C07D 401/14*** *(2006.01)*

(86) International application number:
**PCT/JP2015/084456**

(87) International publication number:
**WO 2016/093255 (16.06.2016 Gazette 2016/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **09.12.2014 JP 2014249349**
**09.12.2014 JP 2014249347**
**09.12.2014 JP 2014249346**

(71) Applicant: **Astellas Pharma Inc.**
**Chuo-ku**
**Tokyo 103-8411 (JP)**

(72) Inventor: **NAGASHIMA, Takeyuki**
**Tokyo 103-8411 (JP)**

(74) Representative: **Hoffmann Eitle**
**Patent- und Rechtsanwälte PartmbB**
**Arabellastraße 30**
**81925 München (DE)**

# (54) PHARMACEUTICAL COMPOSITION HAVING BICYCLIC NITROGEN-CONTAINING AROMATIC HETROCYCLIC AMIDE COMPOUNDS AS ACTIVE INGREDIENT

(57) [Problem] To provide a pharmaceutical composition for treating various cancers in which mitochondrial complex I is involved, in particular, colon cancer, leukemia and/or malignant lymphoma. [Solution] With the invention of creating pharmaceutical compositions for treating various cancers, the inventors confirmed, on the basis of the result of intensive investigation, that pharmaceutical compositions, which contain specific bicyclic nitrogen-containing aromatic heterocyclic amide compounds having an AMPK activation effect and mitochondrial complex I is involved, in particular, colon cancer, leukemia and/or malignant lymphoma; thereby completing the present invention.

EP 3 231 426 A1

## Description

Technical Field

The present invention relates to a pharmaceutical composition for treating various types of cancer in which mitochondrial Complex I is involved, particularly colorectal cancer, leukemia and/or malignant lymphoma, comprising a bicyclic nitrogen-containing aromatic heterocyclic amide compound or a pharmaceutically acceptable salt thereof as an active ingredient.

Background Art

Colorectal cancer is a cancer having high incidence and mortality rates in the world, and the incidence rate is also increasing in Japan every year. Examples of the causes thereof include obesity caused by the Westernization of eating habits, smoking, a lack of physical activity, and the like (Jpn. J. Clin. Oncol. 2013, 43: 685-694). The most effective means for the treatment of colorectal cancer is surgery, but significant progress has been made recently in chemotherapy, radiation therapy, and the like. As a result of large-scale clinical trials performed around Europe and the United States, it became clear that a combination chemotherapy in which various types of anticancer agents are combined is effective for colorectal cancer and contributes to tumor regression and extension of a prognosis (J. Clin. Oncol. 2004, 22: 229-237). Furthermore, in addition to chemotherapy, molecular-targeted drugs such as anti-vascular endothelial growth factor (VEGF) antibody or anti-epidermal growth factor receptor (EGFR) antibody, are being used together with chemotherapy as the primary drug of choice, and further development of the molecular-targeted drugs is expected.

It has been known that mutations in numerous cancer related genes are recognized in colorectal cancer after occurring. It has been reported that in approximately 40% of the mutations, gene mutations occur in molecules involved in the phosphatidylinositol 3-kinase (PI3K) pathway, and for example, mutations may occur in PIK3CA which is the p110$\alpha$ catalytic unit of PI3K (Nature. 2005, 436: 792). When PI3K is activated, Akt, a serine/threonine kinase, is activated by phosphorylation. The mammalian target of rapamycin (mTOR) that is downstream of Akt, is a serine/threonine kinase identified as a target for rapamycin and plays an important role in controlling cell proliferation and survival. The abnormal enhancement of signalling in the PI3K/Akt/mTOR pathway plays an important role in cancer cell proliferation and survival (Oncologist. 2011, 16: 404-414). In addition, it has been reported that in approximately 10% of colorectal cancer, there are mutations in v-raf murine sarcoma viral oncogene homolog b1 (BRAF) genes (Nature. 2002, 417: 949-954). These mutations activate the mitogen-activated protein kinase (MAPK) pathway and induce the abnormal enhancement of expression of MAPK target molecules. It has been also known that there is a crosstalk between PI3K/mTOR pathway and RAF/MAPK pathway (Cell. 2005, 121: 179-193).

In Japan, 90% or more of the patients who have been diagnosed with leukemia are adults aged 20 years or older. The number of people with this disease was about 11,000 per year in 2008 (Foundation for Promotion of Cancer Research, CANCER STATISTICS IN JAPAN-2013). Among these, it is estimated that the number of patients who have been diagnosed with acute myeloid leukemia (AML) exceeds about 3,800 per year (CancerMPact (registered trademark), Japan February 2010, v1.1). 60% to 80% of younger patients with AML achieve complete remission (CR) by using standard treatments, but a disease-free survival rate at 5 years is only 30% to 40% (Hematology Am. Soc. Hematol. Educ. Program 2005, 143). Among patients aged 60 years or older, treatment results become worse, and the CR rate is 40% to 55% and the long-term survival rate is also lower. The remission rate and the overall survival rate depend on many factors such as cytogenetics factors, medical history of a bone marrow disorder (myelodysplastic syndrome and the like), comorbidity, and the like, in addition to age.

In Japan, a combination treatment of cytarabine and anthracycline (daunorubicin or idarubicin) is performed on adult patients with AML under treatment as a standard remission induction therapy (Leukemia treatment manual, 3rd Rev. ed., Nankodo, 2009, 27). Approximately 80% of the patients achieve complete remission by using this treatment, but relapse occurs in approximately 70% of them, and only approximately 30% of the patients remain in remission and survive long-term. In addition, there are approximately 15% of patients who are resistant to a remission induction therapy of the first time. As above, in the treatment of AML, it cannot be said that the current treatment is still sufficient, and aiming for improving a prognosis of the patients with AML, developing a new pharmaceutical agent has significant meaning.

It has been reported that in acute leukemia, the PI3K/Akt/mTOR pathway is abnormally and constantly being activated (Cancer Lett. 2014, 346: 188-196). Furthermore, recently, it has been shown that activities of a mitochondrial respiratory chain decrease in AML cells, and because of this, sensitivity to a respiratory chain inhibitor becomes higher (Blood. 2015, 125: 2120-2130).

Malignant lymphoma is a blood cancer and is a malignant tumor that arises from lymphatic tissues. Lymphoma is roughly divided into Hodgkin's lymphoma (HL) or Hodgkin's disease (HD), and non-Hodgkin's lymphoma (NHL). Furthermore, non-Hodgkin's lymphoma is divided into lymphoma in which B-cells become cancerous (B-cell non-Hodgkin's lymphoma), and lymphoma in which T-cells or NK-cells become cancerous (T/NK-cell non-Hodgkin's lymphoma). It is known that as B-cell non-Hodgkin's lymphoma, there are for example, diffuse large B-cell lymphoma (DLBCL), mantle cell lymphoma (MCL), Burkitt's lymphoma, follicular center lymphoma (FCL), MALT lymphoma (HL), chronic lymphocytic leukemia / small lymphocytic lymphoma (CLL/SLL), and the like, and as T/NK-cell non-Hodgkin's lymphoma, there are for example, adult T-cell lymphoma (ATL), peripheral T-cell lymphoma, lymphoblastic lymphoma, and the like. In addition, in terms of a progression rate, non-Hodgkin's lymphoma is divided into a low-grade group (progresses slowly over a year, often seen in follicular lymphoma and the like), an intermediate-grade group (progresses over a month, often seen in DLBCL and the like), and a high-grade group (progresses rapidly over a week, often seen in Burkitt's lymphoma, lymphoblastic lymphoma, and the like).

Since lymphatic tissues circulate throughout the body, not like other cancers, radiation therapy and chemotherapy are mainly applied instead of extirpation by surgery. It is determined that the point where tumors are no longer detected by treatments is "complete remission", but relapse occurs in many cases, and an object in clinical trials of lymphoma is to improve the complete remission rate and to extend the progression-free survival period.

DLBCL accounts for approximately 30% to 40% of patients with non-Hodgkin's lymphoma, which is the type accounting for the highest rate of lymphoma (Blood. 1997, 89: 3909-3918). From molecular biological analysis, three different subgroups (clusters) are known for the genetic defect of DLBCL, and each one is called oxidative phosphorylation (OXPHOS) type, B cell receptor (BCR) / proliferation type and host response (HR) type. Among these, in OXPHOS type tumors, it is known that genes involved in OXPHOS that is ATP production reaction in the electron transport system of the mitochondria, genes involved in a mitochondrial function, and genes involved in the electron transport chain are highly expressed. Specifically, NADH dehydrogenase complex including mitochondrial Complex I, cytochrome c-cytochrome oxidase complex, ATP synthase, and the like are highly expressed (Blood. 2005, 105: 1851-1861).

It has been reported recently that metformin known as the primary drug of choice of an agent for treating type II diabetes activates adenosine monophosphate (AMP)-activated protein kinase (AMPK), thereby inhibits the proliferation of breast cancer cells, colorectal cancer cells, or AML cells (Cancer Res. 2006, 66: 10269-10273, Cancer Res. 2007, 15: 6745-6752, Blood 2010, 116: 4262-4273). AMPK is a highly preserved serine/threonine kinase, controls energy metabolism in various cells, and monitors changes in the AMP/ATP ratio in cells and responds to it (Annu. Rev. Biochem. 1998, 67: 821-855). The activation of AMPK by metformin is known to be based on the effect of inhibiting mitochondrial Complex I (Diabetes Metab. 2003, 29 (4 Pt 2): 6S88-94). Mitochondrial Complex I is a NADH dehydrogenase located in the mitochondrial inner membrane, and is known as the "entry enzyme" of oxidative phosphorylation in the mitochondria. The inhibition of mitochondrial Complex I leads to the inhibition of oxidative phosphorylation that is ATP production reaction in the electron transport system of the mitochondria. As ATP levels in the cells decrease, AMP/ATP ratio increases, and AMPK is activated by AMP being binding allosterically to AMPK. The activated AMPK inhibits mTOR signaling via phosphorylation of tuberous sclerosis complex 2 (TSC2) that is downstream of the PI3K/Akt pathway (Genes Cells. 2003, 8: 65-79). This is considered to be one of the reasons why metformin inhibits the proliferation of cancer cells (Cancer Res. 2007,67: 10804-10812). Furthermore, it has been reported that the activation of AMPK inhibits the RAF/MAPK pathway, which leads to the inhibition of proliferation of cancer cells having BRAF mutations (Molecular Cell. 2009, 33: 237-247).

As a compound having the effect of inhibiting mitochondrial Complex I, regardless of whether they are natural or unnatural, many types of compounds such as rotenone, pyridaben, bullatacin, piericidin A, capsaicin, fenazaquin, and the like are known. In addition, for example, it has been reported that a compound of Formula (A) below has the effect of inhibiting mitochondrial Complex I and inhibits the proliferation of various types of cancer cells (Patent Document 1).

[Chem. 1]

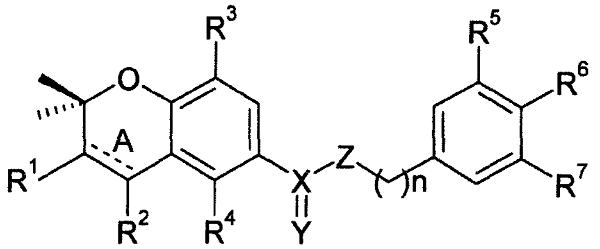

(A)

(refer to the corresponding publication for the meaning of symbols in the formula)

In addition, as a compound having the effect of activating AMPK, it has been reported that for example, a compound of Formulas (B) and (C) below has the effect of activating AMPK, and is useful for treating a metabolic disorder

such as type II diabetes, atherosclerosis, cardiovascular disease, and the like (refer to Patent Document 2 and Patent Document 3, respectively). However, in the documents, there is no specific description that teaches the usefulness for treating cancer and the like.

[Chem. 2]

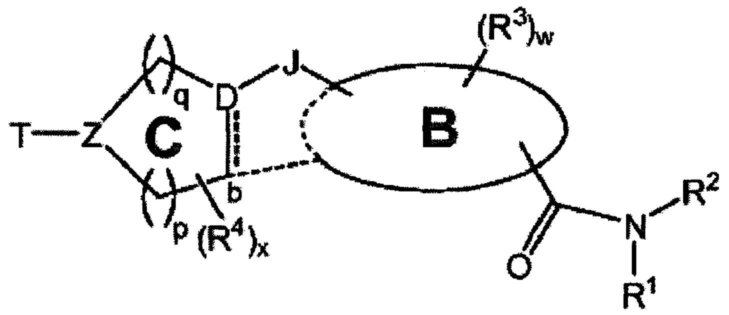

(B)

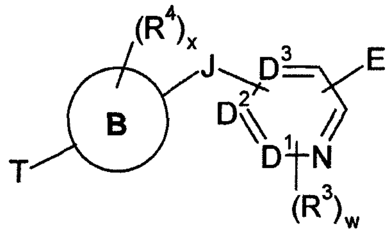

(C)

(refer to the corresponding publication for the meaning of symbols in the formulas)

Related Art Document

Patent Document

Patent Document 1: International Publication No. 02/20008
Patent Document 2: International Publication No. 2009/132136
Patent Document 3: International Publication No. 2012/016217

Disclosure of Invention

Problems to Be Solved by the Invention

A pharmaceutical composition for treating various types of cancer in which mitochondrial Complex I is involved, particularly colorectal cancer, leukemia and/or malignant lymphoma is provided.

Means for Solving the Problems

As a result of intensive examination for creating a pharmaceutical composition for treating various types of cancer, the inventors of the present invention have found that a specific bicyclic nitrogen-containing aromatic heterocyclic amide compound or a pharmaceutically acceptable salt thereof exhibits excellent effect of inhibiting mitochondrial Complex I and the effect of activating AMPK, and that a pharmaceutical composition comprising the compound as an active ingredient, which is disclosed in WO 2014/199933 published after the priority date of the present application, is expected to be used as a pharmaceutical composition for treating cancer selected from colorectal cancer, leukemia, and malignant lymphoma, and in other embodiments, used as a pharmaceutical composition for treating PIK3CA mutation-positive colorectal cancer, a pharmaceutical composition for treating AML, and a pharmaceutical composition for treating cancer selected from DLBCL, and therefore have completed the present invention.

That is, the present invention relates to a pharmaceutical composition for treating cancer selected from colorectal cancer, leukemia, and malignant lymphoma, comprising: a compound selected from:

(5- {1-[(6-methoxypyridin-3-yl)methyl]piperidin-4-yl}-1H-benzimidazol-2-yl) {4-[4-(trifluoromethyl)benzyl]piperazin-1-yl}methanone (hereinafter will be referred to as "Compound A");
(5-{1-[(5-methoxypyrazin-2-yl)methyl]piperidin-4-yl}-1H-benzimidazol-2-yl){4-[4-(trifluoromethyl)benzyl]piperazin-1-yl}methanone (hereinafter will be referred to as "Compound B");
4-({4-[(6-{1-[(5-ethoxypyrazin-2-yl)methyl]piperidin-4-yl}-1-methyl-1H-indol-2-yl)carbonyl]piperazin-1-yl}methyl)benzonitrile (hereinafter will be referred to as "Compound C"); and
4-({4-[(5-{1-[(5-methoxypyrazin-2-yl)methyl]piperidin-4-yl}-1-methyl-1H-indol-2-yl)carbonyl]piperazin-1-yl}methyl)benzonitrile (hereinafter will be referred to as "Compound D"), or a pharmaceutically acceptable salt thereof as an active ingredient.

In addition, the present invention includes an agent for treating cancer selected from colorectal cancer, leukemia, and malignant lymphoma, comprising a compound selected from Compound A, Compound B, Compound C, and Compound D or a pharmaceutically acceptable salt thereof as an active ingredient.

In addition, the present invention relates to the use of a compound selected from Compound A, Compound B, Compound C, and Compound D or a pharmaceutically acceptable salt thereof for the manufacture of a pharmaceutical composition for treating cancer selected from colorectal cancer, leukemia, and malignant lymphoma; the use of a compound selected from Compound A, Compound B, Compound C, and Compound D or a pharmaceutically acceptable salt thereof for treating cancer selected from colorectal cancer, leukemia, and malignant lymphoma; a compound selected from Compound A, Compound B, Compound C, and Compound D or a pharmaceutically acceptable salt thereof for treating colorectal cancer, leukemia, and/or malignant lymphoma; and a method for treating cancer selected from colorectal cancer, leukemia, and malignant lymphoma by administering an effective dose of a compound selected from Compound A, Compound B, Compound C, and Compound D or a pharmaceutically acceptable salt thereof to a subject. The term "subject" refers to humans or any other animals in need of the treatment, and in another embodiment, refers to humans in need of the treatment.

## Effects of the Invention

A compound selected from Compound A, Compound B, Compound C, and Compound D or a pharmaceutically acceptable salt thereof, which are active ingredients of a pharmaceutical composition of the present invention, has the effect of inhibiting mitochondrial Complex I and activating AMPK, and can be used as an active ingredient of a pharmaceutical composition that is for treating cancer selected from colorectal cancer, AML, and DLBCL, in another embodiment, of a pharmaceutical composition for treating cancer selected from PIK3CA mutation-positive colorectal cancer, AML, and DLBCL, and in still another embodiment, of a pharmaceutical composition for treating cancer selected from PIK3CA mutation-positive and BRAF mutation-positive colorectal cancer, AML, and DLBCL.

## Embodiments for Carrying Out the Invention

Hereinafter, the present invention will be described in detail.

Examples of colorectal cancer in which mitochondrial Complex I is involved include colorectal cancer in which the electron transport system of the mitochondria is activated and thereby oxidative phosphorylation is enhanced, PIK3CA mutation-positive colorectal cancer in another embodiment, and PIK3CA mutation-positive and BRAF mutation-positive colorectal cancer in still another embodiment.

Examples of leukemia in which mitochondrial Complex I is involved include leukemia in which the electron transport system of the mitochondria is activated and thereby oxidative phosphorylation is enhanced, AML in another embodiment, and AML in which the PI3K/Akt/mTOR pathway is enhanced in still another embodiment.

Examples of malignant lymphoma in which mitochondrial Complex I is involved include malignant lymphoma in which the electron transport system of the mitochondria is activated and thereby oxidative phosphorylation is enhanced, DLBCL in another embodiment, and OXPHOS-DLBCL in still another embodiment.

In the present specification, "a pharmaceutically acceptable salt of a compound selected from Compound A, Compound B, Compound C, and Compound D" means an acid addition salt of Compound A, Compound B, Compound C, or Compound D. Specific examples of the acid addition salt include the salts with inorganic acids such as hydrochloric acid, hydrobromic acid, hydroiodic acid, sulfuric acid, nitric acid, phosphoric acid and the like, and organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid (mesylic acid), ethanesulfonic acid, benzenesulfonic acid, p-toluenesulfonic acid (tosylic acid), aspartic acid, glutamic acid, and the like.

Examples of "a compound selected from Compound A, Compound B, Compound C, and Compound D" include various solvates of Compound A, Compound B, Compound C, or Compound D, and specifically include a hydrate or an

ethanol solvate. Furthermore, "a pharmaceutically acceptable salt" includes an acid addition salt of these solvates.

In addition, examples of "a compound selected from Compound A, Compound B, Compound C, and Compound D or a pharmaceutically acceptable salt thereof" include freebase in which a salt is not formed, that is, Compound A, Compound B, Compound C, or Compound D in a certain embodiment, Compound A in another embodiment, Compound B in still another embodiment, and Compound C in still further another embodiment, and Compound D in still further another embodiment. Furthermore, a tosylate salt of Compound A, Compound B, Compound C, or Compound D is included in another embodiment, a ditosylate salt of Compound A in still another embodiment, a ditosylate salt of Compound B in still further another embodiment, a ditosylate salt of Compound C in still further another embodiment, and a ditosylate salt of Compound D in still further another embodiment.

The embodiments of the present invention are presented as below.

(1-1) A pharmaceutical composition for treating cancer selected from colorectal cancer, leukemia in which mitochondrial Complex I is involved, and malignant lymphoma in which mitochondrial Complex I is involved, comprising a compound selected from Compound A, Compound B, Compound C, and Compound D or a pharmaceutically acceptable salt thereof as an active ingredient. In another embodiment, a pharmaceutical composition for treating cancer selected from colorectal cancer, AML, and DLBCL, comprising a compound selected from Compound A, Compound B, Compound C, and Compound D or a pharmaceutically acceptable salt thereof as an active ingredient. In still another embodiment, a pharmaceutical composition for treating cancer selected from colorectal cancer, leukemia in which mitochondrial Complex I is involved, and malignant lymphoma in which mitochondrial Complex I is involved, comprising a ditosylate salt of a compound selected from Compound A, Compound B, Compound C, and Compound D as an active ingredient. In still further another embodiment, a pharmaceutical composition for treating cancer selected from colorectal cancer, AML, and DLBCL, comprising a ditosylate salt of a compound selected from Compound A, Compound B, Compound C, and Compound D as an active ingredient.

(1-2) The use of a compound selected from Compound A, Compound B, Compound C, and Compound D or a pharmaceutically acceptable salt thereof for the manufacture of a pharmaceutical composition for treating cancer selected from colorectal cancer, leukemia in which mitochondrial Complex I is involved, and malignant lymphoma in which mitochondrial Complex I is involved. In another embodiment, the use of a compound selected from Compound A, Compound B, Compound C, and Compound D or a pharmaceutically acceptable salt thereof for the manufacture of a pharmaceutical composition for treating cancer selected from colorectal cancer, AML, and DLBCL. In still another embodiment, the use of a ditosylate salt of a compound selected from Compound A, Compound B, Compound C, and Compound D for the manufacture of a pharmaceutical composition for treating cancer selected from colorectal cancer, leukemia in which mitochondrial Complex I is involved, and malignant lymphoma in which mitochondrial Complex I is involved. In still further another embodiment, the use of a ditosylate salt of a compound selected from Compound A, Compound B, Compound C, and Compound D for the manufacture of a pharmaceutical composition for treating cancer selected from colorectal cancer, AML, and DLBCL.

(1-3) The use of a compound selected from Compound A, Compound B, Compound C, and Compound D or a pharmaceutically acceptable salt thereof for treating cancer selected from colorectal cancer, leukemia in which mitochondrial Complex I is involved, and malignant lymphoma in which mitochondrial Complex I is involved. In another embodiment, the use of a compound selected from Compound A, Compound B, Compound C, and Compound D or a pharmaceutically acceptable salt thereof for treating cancer selected from colorectal cancer, AML, and DLBCL. In still another embodiment, the use of a ditosylate salt of a compound selected from Compound A, Compound B, Compound C, and Compound D for treating cancer selected from colorectal cancer, leukemia in which mitochondrial Complex I is involved, and malignant lymphoma in which mitochondrial Complex I is involved. In still further another embodiment, the use of a ditosylate salt of a compound selected from Compound A, Compound B, Compound C, and Compound D for treating cancer selected from colorectal cancer, AML, and DLBCL.

(1-4) A compound selected from Compound A, Compound B, Compound C, and Compound D or a pharmaceutically acceptable salt thereof for treating cancer selected from colorectal cancer, leukemia in which mitochondrial Complex I is involved, and malignant lymphoma in which mitochondrial Complex I is involved. In another embodiment, a compound selected from Compound A, Compound B, Compound C, and Compound D or a pharmaceutically acceptable salt thereof for treating cancer selected from colorectal cancer, AML, and DLBCL. In still another embodiment, a ditosylate salt of a compound selected from Compound A, Compound B, Compound C, and Compound D for treating cancer selected from colorectal cancer, leukemia in which mitochondrial Complex I is involved, and malignant lymphoma in which mitochondrial Complex I is involved. In still further another embodiment, a ditosylate salt of a compound selected from Compound A, Compound B, Compound C, and Compound D for treating cancer selected from colorectal cancer, AML, and DLBCL.

(1-5) A method for treating cancer selected from colorectal cancer, leukemia in which mitochondrial Complex I is involved, and malignant lymphoma in which mitochondrial Complex I is involved by administering an effective dose of a compound selected from Compound A, Compound B, Compound C, and Compound D or a pharmaceutically

acceptable salt thereof to a subject. In another embodiment, a method for treating cancer selected from colorectal cancer, AML, and DLBCL by administering an effective dose of a compound selected from Compound A, Compound B, Compound C, and Compound D or a pharmaceutically acceptable salt thereof to a subject. In still another embodiment, a method for treating cancer selected from colorectal cancer, leukemia in which mitochondrial Complex I is involved, and malignant lymphoma in which mitochondrial Complex I is involved by administering an effective dose of a ditosylate salt of a compound selected from Compound A, Compound B, Compound C, and Compound D to a subject. In still further another embodiment, a method for treating cancer selected from colorectal cancer, AML, and DLBCL by administering an effective dose of a ditosylate salt of a compound selected from Compound A, Compound B, Compound C, and Compound D to a subject.

(2-1) A pharmaceutical composition for treating colorectal cancer, comprising a compound selected from Compound A, Compound B, Compound C, and Compound D or a pharmaceutically acceptable salt thereof as an active ingredient. In another embodiment, a pharmaceutical composition for treating PIK3CA mutation-positive colorectal cancer, comprising a compound selected from Compound A, Compound B, Compound C, and Compound D or a pharmaceutically acceptable salt thereof as an active ingredient. In still another embodiment, a pharmaceutical composition for treating PIK3CA mutation-positive and BRAF mutation-positive colorectal cancer, comprising a compound selected from Compound A, Compound B, Compound C, and Compound D or a pharmaceutically acceptable salt thereof as an active ingredient. In still further another embodiment, a pharmaceutical composition for treating colorectal cancer, comprising a ditosylate salt of a compound selected from Compound A, Compound B, Compound C, and Compound D as an active ingredient. In still further another embodiment, a pharmaceutical composition for treating PIK3CA mutation-positive colorectal cancer, comprising a ditosylate salt of a compound selected from Compound A, Compound B, Compound C, and Compound D as an active ingredient. In still further another embodiment, a pharmaceutical composition for treating PIK3CA mutation-positive and BRAF mutation-positive colorectal cancer, comprising a ditosylate salt of a compound selected from Compound A, Compound B, Compound C, and Compound D as an active ingredient.

(2-2) The use of a compound selected from Compound A, Compound B, Compound C, and Compound D or a pharmaceutically acceptable salt thereof for the manufacture of a pharmaceutical composition for treating colorectal cancer. In another embodiment, the use of a compound selected from Compound A, Compound B, Compound C, and Compound D or a pharmaceutically acceptable salt thereof for the manufacture of a pharmaceutical composition for treating PIK3CA mutation-positive colorectal cancer. In still another embodiment, the use of a compound selected from Compound A, Compound B, Compound C, and Compound D or a pharmaceutically acceptable salt thereof for the manufacture of a pharmaceutical composition for treating PIK3CA mutation-positive and BRAF mutation-positive colorectal cancer. In still further another embodiment, the use of a ditosylate salt of a compound selected from Compound A, Compound B, Compound C, and Compound D for the manufacture of a pharmaceutical composition for treating colorectal cancer. In still further another embodiment, the use of a ditosylate salt of a compound selected from Compound A, Compound B, Compound C, and Compound D for the manufacture of a pharmaceutical composition for treating PIK3CA mutation-positive colorectal cancer. In still further another embodiment, the use of a ditosylate salt of a compound selected from Compound A, Compound B, Compound C, and Compound D for the manufacture of a pharmaceutical composition for treating PIK3CA mutation-positive and BRAF mutation-positive colorectal cancer.

(2-3) The use of a compound selected from Compound A, Compound B, Compound C, and Compound D or a pharmaceutically acceptable salt thereof for treating colorectal cancer. In another embodiment, the use of a compound selected from Compound A, Compound B, Compound C, and Compound D or a pharmaceutically acceptable salt thereof for treating PIK3CA mutation-positive colorectal cancer. In still another embodiment, the use of a compound selected from Compound A, Compound B, Compound C, and Compound D or a pharmaceutically acceptable salt thereof for treating PIK3CA mutation-positive and BRAF mutation-positive colorectal cancer. In still further another embodiment, the use of a ditosylate salt of a compound selected from Compound A, Compound B, Compound C, and Compound D for treating colorectal cancer. In still further another embodiment, the use of a ditosylate salt of a compound selected from Compound A, Compound B, Compound C, and Compound D for treating PIK3CA mutation-positive colorectal cancer. In still further another embodiment, the use of a ditosylate salt of a compound selected from Compound A, Compound B, Compound C, and Compound D for treating PIK3CA mutation-positive and BRAF mutation-positive colorectal cancer.

(2-4) A compound selected from Compound A, Compound B, Compound C, and Compound D or a pharmaceutically acceptable salt thereof for treating colorectal cancer. In another embodiment, a compound selected from Compound A, Compound B, Compound C, and Compound D or a pharmaceutically acceptable salt thereof for treating PIK3CA mutation-positive colorectal cancer. In still another embodiment, a compound selected from Compound A, Compound

B, Compound C, and Compound D or a pharmaceutically acceptable salt thereof for treating PIK3CA mutation-positive and BRAF mutation-positive colorectal cancer. In still further another embodiment, a ditosylate salt of a compound selected from Compound A, Compound B, Compound C, and Compound D for treating colorectal cancer. In still further another embodiment, a ditosylate salt of a compound selected from Compound A, Compound B, Compound C, and Compound D for treating PIK3CA mutation-positive colorectal cancer. In still further another embodiment, a ditosylate salt of a compound selected from Compound A, Compound B, Compound C, and Compound D for treating PIK3CA mutation-positive and BRAF mutation-positive colorectal cancer.

(2-5) A method for treating colorectal cancer by administering an effective dose of a compound selected from Compound A, Compound B, Compound C, and Compound D or a pharmaceutically acceptable salt thereof to a subject. In another embodiment, a method for treating PIK3CA mutation-positive colorectal cancer by administering an effective dose of a compound selected from Compound A, Compound B, Compound C, and Compound D or a pharmaceutically acceptable salt thereof to a subject. In still another embodiment, a method for treating PIK3CA mutation-positive and BRAF mutation-positive colorectal cancer by administering an effective dose of a compound selected from Compound A, Compound B, Compound C, and Compound D or a pharmaceutically acceptable salt thereof to a subject. In still further another embodiment, a method for treating colorectal cancer by administering an effective dose of a ditosylate salt of a compound selected from Compound A, Compound B, Compound C, and Compound D to a subject. In still further another embodiment, a method for treating PIK3CA mutation-positive colorectal cancer by administering an effective dose of a ditosylate salt of a compound selected from Compound A, Compound B, Compound C, and Compound D to a subject. In still further another embodiment, a method for treating PIK3CA mutation-positive and BRAF mutation-positive colorectal cancer by administering an effective dose of a ditosylate salt of a compound selected from Compound A, Compound B, Compound C, and Compound D to a subject.

(3-1) A pharmaceutical composition for treating leukemia in which mitochondrial Complex I is involved, comprising a compound selected from Compound A, Compound B, Compound C, and Compound D or a pharmaceutically acceptable salt thereof as an active ingredient. In another embodiment, a pharmaceutical composition for treating AML, comprising a compound selected from Compound A, Compound B, Compound C, and Compound D or a pharmaceutically acceptable salt thereof as an active ingredient. In still another embodiment, a pharmaceutical composition for treating AML in which the PI3K/Akt/mTOR pathway is enhanced, comprising a compound selected from Compound A, Compound B, Compound C, and Compound D or a pharmaceutically acceptable salt thereof as an active ingredient. In still further another embodiment, a pharmaceutical composition for treating leukemia in which mitochondrial Complex I is involved, comprising a ditosylate salt of a compound selected from Compound A, Compound B, Compound C, and Compound D as an active ingredient. In still further another embodiment, a pharmaceutical composition for treating AML, comprising a ditosylate salt of a compound selected from Compound A, Compound B, Compound C, and Compound D as an active ingredient. In still further another embodiment, a pharmaceutical composition for treating AML in which the PI3K/Akt/mTOR pathway is enhanced, comprising a ditosylate salt of a compound selected from Compound A, Compound B, Compound C, and Compound D as an active ingredient.

(3-2) The use of a compound selected from Compound A, Compound B, Compound C, and Compound D or a pharmaceutically acceptable salt thereof for the manufacture of a pharmaceutical composition for treating leukemia in which mitochondrial Complex I is involved. In another embodiment, the use of a compound selected from Compound A, Compound B, Compound C, and Compound D or a pharmaceutically acceptable salt thereof for the manufacture of a pharmaceutical composition for treating AML. In still another embodiment, the use of a compound selected from Compound A, Compound B, Compound C, and Compound D or a pharmaceutically acceptable salt thereof for the manufacture of a pharmaceutical composition for treating AML in which the PI3K/Akt/mTOR pathway is enhanced. In still further another embodiment, the use of a ditosylate salt of a compound selected from Compound A, Compound B, Compound C, and Compound D for the manufacture of a pharmaceutical composition for treating leukemia in which mitochondrial Complex I is involved. In still further another embodiment, the use of a ditosylate salt of a compound selected from Compound A, Compound B, Compound C, and Compound D for the manufacture of a pharmaceutical composition for treating AML. In still further another embodiment, the use of a ditosylate salt of a compound selected from Compound A, Compound B, Compound C, and Compound D for the manufacture of a pharmaceutical composition for treating AML in which the PI3K/Akt/mTOR pathway is enhanced.

(3-3) The use of a compound selected from Compound A, Compound B, Compound C, and Compound D or a pharmaceutically acceptable salt thereof for treating leukemia in which mitochondrial Complex I is involved. In another embodiment, the use of a compound selected from Compound A, Compound B, Compound C, and Compound D or a pharmaceutically acceptable salt thereof for treating AML. In still another embodiment, the use of a compound selected from Compound A, Compound B, Compound C, and Compound D or a pharmaceutically acceptable salt thereof for treating AML in which the PI3K/Akt/mTOR pathway is enhanced. In still further another embodiment, the use of a ditosylate salt of a compound selected from Compound A, Compound B, Compound C, and Compound D for treating leukemia in which mitochondrial Complex I is involved. In still further another embodiment, the use of a ditosylate salt of a compound selected from Compound A, Compound B, Compound C, and Compound D for treating AML. In still further another embodiment, the use of a ditosylate salt of a compound selected from Compound A, Compound B, Compound C, and Compound D for treating AML in which the PI3K/Akt/mTOR pathway is enhanced.

(3-4) A compound selected from Compound A, Compound B, Compound C, and Compound D or a pharmaceutically acceptable salt thereof for treating leukemia in which mitochondrial Complex I is involved. In another embodiment, a compound selected from Compound A, Compound B, Compound C, and Compound D or a pharmaceutically acceptable salt thereof for treating AML. In still another embodiment, a compound selected from Compound A, Compound B, Compound C, and Compound D or a pharmaceutically acceptable salt thereof for treating AML in which the PI3K/Akt/mTOR pathway is enhanced. In still further another embodiment, a ditosylate salt of a compound selected from Compound A, Compound B, Compound C, and Compound D for treating leukemia in which mitochondrial Complex I is involved. In still further another embodiment, a ditosylate salt of a compound selected from Compound A, Compound B, Compound C, and Compound D for treating AML. In still further another embodiment, a ditosylate salt of a compound selected from Compound A, Compound B, Compound C, and Compound D for treating AML in which the PI3K/Akt/mTOR pathway is enhanced.

(3-5) A method for treating leukemia in which mitochondrial Complex I is involved by administering an effective dose of a compound selected from Compound A, Compound B, Compound C, and Compound D or a pharmaceutically acceptable salt thereof to a subject. In another embodiment, a method for treating AML by administering an effective dose of a compound selected from Compound A, Compound B, Compound C, and Compound D or a pharmaceutically acceptable salt thereof to a subject. In still another embodiment, a method for treating AML in which the PI3K/Akt/mTOR pathway is enhanced by administering an effective dose of a compound selected from Compound A, Compound B, Compound C, and Compound D or a pharmaceutically acceptable salt thereof to a subject. In still further another embodiment, a method for treating leukemia in which mitochondrial Complex I is involved by administering an effective dose of a ditosylate salt of a compound selected from Compound A, Compound B, Compound C, and Compound D to a subject. In still further another embodiment, a method for treating AML by administering an effective dose of a ditosylate salt of a compound selected from Compound A, Compound B, Compound C, and Compound D to a subject. In still further another embodiment, a method for treating AML in which the PI3K/Akt/mTOR pathway is enhanced by administering an effective dose of a ditosylate salt of a compound selected from Compound A, Compound B, Compound C, and Compound D to a subject.

(4-1) A pharmaceutical composition for treating malignant lymphoma in which mitochondrial Complex I is involved, comprising a compound selected from Compound A, Compound B, Compound C, and Compound D or a pharmaceutically acceptable salt thereof as an active ingredient. In another embodiment, a pharmaceutical composition for treating DLBCL, comprising a compound selected from Compound A, Compound B, Compound C, and Compound D or a pharmaceutically acceptable salt thereof as an active ingredient. In still another embodiment, a pharmaceutical composition for treating OXPHOS-type DLBCL, comprising a compound selected from Compound A, Compound B, Compound C, and Compound D or a pharmaceutically acceptable salt thereof as an active ingredient. In still further another embodiment, a pharmaceutical composition for treating malignant lymphoma in which mitochondrial Complex I is involved, comprising a ditosylate salt of a compound selected from Compound A, Compound B, Compound C, and Compound D as an active ingredient. In still further another embodiment, a pharmaceutical composition for treating DLBCL, comprising a ditosylate salt of a compound selected from Compound A, Compound B, Compound C, and Compound D as an active ingredient. In still further another embodiment, a pharmaceutical composition for treating OXPHOS-type DLBCL, comprising a ditosylate salt of a compound selected from Compound A, Compound B, Compound C, and Compound D as an active ingredient.

(4-2) The use of a compound selected from Compound A, Compound B, Compound C, and Compound D or a pharmaceutically acceptable salt thereof for the manufacture of a pharmaceutical composition for treating malignant lymphoma in which mitochondrial Complex I is involved. In another embodiment, the use of a compound selected from Compound A, Compound B, Compound C, and Compound D or a pharmaceutically acceptable salt thereof for the manufacture of a pharmaceutical composition for treating DLBCL. In still another embodiment, the use of a compound selected from Compound A, Compound B, Compound C, and Compound D or a pharmaceutically acceptable salt thereof for the manufacture of a pharmaceutical composition for treating OXPHOS-type DLBCL. In still further another embodiment, the use of a ditosylate salt of a compound selected from Compound A, Compound B, Compound C, and Compound D for the manufacture of a pharmaceutical composition for treating malignant

lymphoma in which mitochondrial Complex I is involved. In still further another embodiment, the use of a ditosylate salt of a compound selected from Compound A, Compound B, Compound C, and Compound D for the manufacture of a pharmaceutical composition for treating DLBCL. In still further another embodiment, the use of a ditosylate salt of a compound selected from Compound A, Compound B, Compound C, and Compound D for the manufacture of a pharmaceutical composition for treating OXPHOS-type DLBCL.

(4-3) The use of a compound selected from Compound A, Compound B, Compound C, and Compound D or a pharmaceutically acceptable salt thereof for treating malignant lymphoma in which mitochondrial Complex I is involved. In another embodiment, the use of a compound selected from Compound A, Compound B, Compound C, and Compound D or a pharmaceutically acceptable salt thereof for treating DLBCL. In still another embodiment, the use of a compound selected from Compound A, Compound B, Compound C, and Compound D or a pharmaceutically acceptable salt thereof for treating OXPHOS-type DLBCL. In still further another embodiment, the use of a ditosylate salt of a compound selected from Compound A, Compound B, Compound C, and Compound D for treating malignant lymphoma in which mitochondrial Complex I is involved. In still further another embodiment, the use of a ditosylate salt of a compound selected from Compound A, Compound B, Compound C, and Compound D for treating DLBCL. In still further another embodiment, the use of a ditosylate salt of a compound selected from Compound A, Compound B, Compound C, and Compound D for treating OXPHOS-type DLBCL.

(4-4) A compound selected from Compound A, Compound B, Compound C, and Compound D or a pharmaceutically acceptable salt thereof for treating malignant lymphoma in which mitochondrial Complex I is involved. In another embodiment, a compound selected from Compound A, Compound B, Compound C, and Compound D or a pharmaceutically acceptable salt thereof for treating DLBCL. In still another embodiment, a compound selected from Compound A, Compound B, Compound C, and Compound D or a pharmaceutically acceptable salt thereof for treating OXPHOS-type DLBCL. In still further another embodiment, a ditosylate salt of a compound selected from Compound A, Compound B, Compound C, and Compound D for treating malignant lymphoma in which mitochondrial Complex I is involved. In still further another embodiment, a ditosylate salt of a compound selected from Compound A, Compound B, Compound C, and Compound D for treating DLBCL. In still further another embodiment, a ditosylate salt of a compound selected from Compound A, Compound B, Compound C, and Compound D for treating OXPHOS-type DLBCL.

(4-5) A method for treating malignant lymphoma in which mitochondrial Complex I is involved by administering an effective dose of a compound selected from Compound A, Compound B, Compound C, and Compound D or a pharmaceutically acceptable salt thereof to a subject. In another embodiment, a method for treating DLBCL by administering an effective dose of a compound selected from Compound A, Compound B, Compound C, and Compound D or a pharmaceutically acceptable salt thereof to a subject. In still another embodiment, a method for treating OXPHOS-type DLBCL by administering an effective dose of a compound selected from Compound A, Compound B, Compound C, and Compound D or a pharmaceutically acceptable salt thereof to a subject. In still further another embodiment, a method for treating malignant lymphoma in which mitochondrial Complex I is involved by administering an effective dose of a ditosylate salt of a compound selected from Compound A, Compound B, Compound C, and Compound D to a subject. In still further another embodiment, a method for treating DLBCL by administering an effective dose of a ditosylate salt of a compound selected from Compound A, Compound B, Compound C, and Compound D to a subject. In still further another embodiment, a method for treating OXPHOS-type DLBCL by administering an effective dose of a ditosylate salt of a compound selected from Compound A, Compound B, Compound C, and Compound D to a subject.

Pharmacological effects of pharmaceutical composition of the present invention are confirmed by Test Example below. In Test Example below, a ditosylate salt of Compound A (hereinafter will be referred to as Compound A1. Example Number 1 (Ex. 1) in Table 15 described below), a ditosylate salt of Compound B (hereinafter will be referred to as Compound B1. Example Number 2 (Ex. 2) in Table 15 described below), a ditosylate salt of Compound C (hereinafter will be referred to as Compound C 1. Example Number 3 (Ex. 3) in Table 15 described below), and a ditosylate salt of Compound D (hereinafter will be referred to as Compound D1. Example Number 4 (Ex. 4) in Table 15 described below) were used as a test compound. In each Test Example, the concentration of Compounds A1, B1, C1, and D1 is described in terms of the concentration of each freebase.

Test Example 1 Evaluation on Effect of Inhibiting Human Mitochondrial Complex I

Mitochondria were extracted from MDA-MB-453 tumor that is Human PIK3CA mutation-positive breast cancer, and the activity of inhibiting Complex I by Compounds A1, B1, C1, and D1 was evaluated.
PIK3CA mutation-positive breast cancer refers to breast cancer having mutations in PIK3CA, a gene name of p110α which is the catalytic subunit of PI3K, among mutations in phosphatidylinositol 3-kinase (PI3K) pathway genes.
In addition, MDA-MB-453 cells used in this Test Example and the next Test Example 2 were obtained from the

American Type Culture Collection (hereinafter will be referred to as ATCC).

4 week old male nude mice (CHARLES RIVER LABORATORIES JAPAN, INC.) were tumor-bearing MDA-MB-453 cells derived from human PIK3CA mutation-positive breast cancer under their skin, and after MDA-MB-453 tumor reached a certain size, it was extracted. A solution of 9 times the tumor weight for extraction of mitochondria (0.275 M Sucrose, 2.2 mM EDTA, 11 mM Tris/HCl, pH 7.5, Complete-EDTA-free (Roche Diagnostics)) was added thereto, and then the tumor was crushed. The centrifugation was performed at 600 x g for 10 minutes at 4°C, the supernatant was obtained, and then the centrifugation was performed at 14,000 x g for 10 minutes at 4°C, and therefore pellets were obtained. The pellets were suspended in 10 mM Tris/HCl pH 7.5 of 5 times the weight of the extracted tumor, and therefore a suspension of human mitochondria was obtained.

Next, 25 or 50 μl of the suspension of human mitochondria was added per 1 ml of a liquid for measuring Complex I activity (25 mM potassium phosphate, pH 7.6, 0.35% Bovine Serum Albumin (BSA), 60 μM 2,6-dichlorophenol-indophenol, 70 μM decylubiquinone, 1 μM antimycin). After the solution was collected and added to a 96 or 384 well plate, a test compound was added to an arbitrary range from the final concentration of 10,000 nM to the final concentration of 0.3 nM. As a negative control, dimethylsulfoxide (DMSO) that is a solvent for the test compound was added to the final concentration of 1%, and as a positive control, rotenone that is a Complex I inhibitor was added to the final concentration of 1 μM. Furthermore, NADH was added to each well to the final concentration of 0.2 or 0.5 mM, and the change in absorbance at a wavelength of 600 nm was measured by using the SpectraMax (Molecular Devices, LLC.) set to 37°C in advance. Signal values in a DMSO treatment were set to a top value, and signal values in a rotenone 1 μM treatment were set to a bottom value. Fluctuations in the signals were calculated within a range where the reaction was linear, and 50% inhibition values ($IC_{50}$) were calculated by a nonlinear regression analysis method using a Sigmoid Emax model. The result of Test Compounds A1, B1, C1, and D1 is shown in Table 1. Compounds A1 to D1 were produced in Examples 1 to 4 described below (the same shall apply hereinafter).

[Table 1]

| Test Compound | $IC_{50}$ (nM) |
|---|---|
| A1 | 41 |
| B1 | 75 |
| C1 | 22 |
| D1 | 120 |

Test Example 2 Evaluation on Effect of Activating AMPK

Phosphorylation of 79th serine (Ser79) of Acetyl-CoA Carboxylase (ACC) which is a substrate of AMPK was measured using Cell ELISA, and thereby the effect of activating AMPK by Compounds A1, B1, C1, and D1 was evaluated.

Each 36 μl of MDA-MB-453 cells was seeded in Leibovitz's L-15 medium including 10% fetal bovine serum (Life Technologies Corporation) in a 384 well plate, so that the cells became 15,000 cells per well, and the cells were cultured overnight at 37°C in the absence of $CO_2$. On the following day, Test Compounds A1, B1, C1, and D1, and DMSO which is a solvent for the test compounds as a negative control were diluted to a 10-fold concentration of a final concentration with a fresh medium, and 4 μl of the resultant product was added to each well (the test compounds had 10 steps in a final concentration from 10,000 nM to 0.3 nM, and DMSO had a final concentration of 0.1 %). After that, the cells were cultured at 37°C for 2 hours in the absence of $CO_2$. After the cultivation, 20 μl of a 40% glyoxal solution (Nacalai Tesque, INC.) was added to each well, and then the cells were left to stand at room temperature for 30 minutes to be fixed. Thereafter, the supernatant was removed by centrifuging the plate (at 800 rpm for 8 seconds by using the Ecospin of C.A.N. Hereinafter the centrifugation was performed under the same conditions unless otherwise specified), and 20 μl of 0.1% Triton X-100-containing Phosphate-Buffered Saline (PBS) was added to each well, and then left to stand at room temperature for 10 minutes. The 0.1% Triton X-100-containing PBS was removed by centrifugation, and 20 μl of a blocking solution (Odyssey Blocking Buffer manufactured by LI-COR Biosciences, Inc.) was added to each well, and then left to stand at room temperature for 1 hour. The blocking solution was removed by centrifugation, and 10 μl of a blocking solution in which the amount of a phosphorylation antibody (manufactured by Cell Signaling Technology, Inc.) of ACC Ser79 as a primary antibody is 1/500 with respect to the undiluted solution, was added to each well, and then left to stand at 4°C overnight. On the following day, the reaction liquid was removed by centrifuging the plate, and 25 μl of 0.05% Tween-20-containing Tris-Buffered Saline (TBS) (manufacture by Thermo Scientific; used in 1x in which 20xTBS Tween-20 was diluted with ionexchange water) was added to each well, and then each well was washed by centrifugal removal. The washing was performed for a total of 3 times. After washing, 10 μl of a blocking solution in which the amount of IRDye (registerd trademark) 800CW Goat anti-Rabbit IgG (manufactured by LI-COR

Biosciences, Inc.) as a secondary antibody is 1/1000 with respect to the undiluted solution, was added to each well, and then left to stand at room temperature for 1 hour. The reaction liquid was removed by centrifuging the plate, and then each well was washed 3 times with 0.05% Tween-20-containing TBS in the same manner as after the primary antibody reaction. After the washing solution was removed, without change, the plate was air-dried at room temperature for 3 hours or longer and signals were measured by Aerius (manufactured by LI-COR Biosciences, Inc.). Signal values in a DMSO treatment were set to a bottom value, and signal values when reaching a plateau were set to a top value, and 50% activation values ($EC_{50}$) were calculated by a nonlinear regression analysis method using a Sigmoid Emax model. The result of Test Compounds A1, B1, C1, and D1 is shown in Table 2.

[Table 2]

| Test Compound | $EC_{50}$ (nM) |
|---|---|
| A1 | 24 |
| B1 | 8.3 |
| C1 | 1.8 |
| D1 | 3.3 |

Test Example 3 Anti-Tumor Test 1 on Mice Tumor-bearing Human Colorectal Cancer-derived Cell

5 to 6 week old male Balb/c nude mice (CHARLES RIVER LABORATORIES JAPAN, INC.) were injected and implanted, under the skin on the back, with 1.5 x $10^6$ PIK3CA mutation-positive and BRAF mutation-positive RKO cells, or 3 x $10^6$ PIK3CA mutation-positive Colo201 cells, both derived from human colorectal cancer suspended to a PBS solution or a mixed solution of PBS and Matrigel (registered trademark) in 1:1. Dividing into groups was performed when the tumor volume reached 100 to 300 $mm^3$, and administering of the test compounds was started. The test was performed on each of the 5 mice in a solvent group and a compound administered group. By oral administration, 6% cyclodextrin aqueous solution for the solvent group, and 6% cyclodextrin aqueous solution in which the test compound was mixed by 8 mg/kg for the compound administered group were administered. The administration was performed once a day for 10 days (RKO) or 14 days (Colo201), and the body weight and the tumor diameter were measured twice a week. For calculation of the tumor volume, the formula below was used.

$$[\text{Tumor volume (mm}^3)]=[\text{major axis of tumor (mm)}]\text{x}[\text{minor axis of tumor (mm)}]^2\text{x}0.5$$

The inhibition rate of tumor growth was calculated from the average value of the tumor volume according to the formula below.

$$\text{Inhibition rate of tumor growth (\%)}=(1-\text{average of tumor volume growth in each group/average of tumor volume growth in solvent group})\text{x}100$$

The anti-tumor effect of Compound A1 on the final measuring day is shown in Table 3.

RKO cells and Colo201 cells derived from human colorectal cancer can be purchased from, for example, ATCC or the like.

[Table 3]

| Cell | Inhibition rate |
|---|---|
| RKO | 67% |
| Colo201 | 60% |

Test Example 4 Anti-Tumor Test 2 on Mice Tumor-bearing Human Colorectal Cancer-derived Cell

4 to 5 week old male Balb/c nude mice (CHARLES RIVER LABORATORIES JAPAN, INC.) were injected and implanted, under the skin on the back, with 3 x $10^6$ PIK3CA mutation-positive and BRAF mutation-positive RKO cells, or 3 x $10^6$ PIK3CA mutation-positive Colo201 cells, both derived from human colorectal cancer suspended to a mixed

solution of PBS and Matrigel (registered trademark) in 1:1. Dividing into groups was performed when the tumor volume reached 100 to 250 $mm^3$, and administering of the test compounds was started. The test was performed on each of the 5 mice in the solvent group and the compound administered group. By oral administration, 6% cyclodextrin aqueous solution for the solvent group, and 6% cyclodextrin aqueous solution in which the test compound was mixed by the dosage shown in Table 4 for the compound administered group were administered. The administration was performed once a day for 11 days (RKO) or 21 days (Colo201), and the body weight and the tumor diameter were measured twice a week. For calculation of the tumor volume, the formula below was used.

$$[\text{Tumor volume } (mm^3)]=[\text{major axis of tumor } (mm)]x[\text{minor axis of tumor } (mm)]^2x0.5$$

The inhibition rate of tumor growth was calculated from the average value of the tumor volume according to the formula below.

Inhibition rate of tumor growth (%)=(1 - average of tumor volume growth in each group/average of tumor volume growth in solvent group)x100

The anti-tumor effect of Test Compounds A1, B1, C1, and D1 on the final measuring day is shown in Table 4.

RKO cells and Colo201 cells were purchased from ATCC.

[Table 4]

| Test Compound | Dosage (mg/kg) | Inhibition rate RKO | Inhibition rate Colo201 |
|---|---|---|---|
| A1 | 8 | 48% | 53% |
| B1 | 8 | 55% | 52% |
| C1 | 1 | 58% | 54% |
| D1 | 1 | 13% | 45% |

Test Example 5 Anti-Tumor Test 1 on Tumor-bearing Mice transplanted with Human AML-derived KG-1 Cell

4 week old male Balb/c nude mice (CHARLES RIVER LABORATORIES JAPAN, INC.) were injected and implanted, under the skin on the back, with 3 x $10^6$ KG-1 cells, suspended to a mixed solution of PBS and Matrigel (registered trademark) in 1:1. After 16 days of implantation, administering of the test compounds was started. The test was performed on each of the 5 mice in the solvent group (6% cyclodextrin aqueous solution) and the test compound administered group (Test Compound A1 was mixed to the solvent group by a dosage of 2, 4, or 8 mg/kg). The administration was performed once a day for 14 days by oral administration, and the body weight and the tumor diameter were measured twice a week. For calculation of the tumor volume, the formula below was used.

$$[\text{Tumor volume } (mm^3)]=[\text{major axis of tumor } (mm)]x[\text{minor axis of tumor } (mm)]^2x0.5$$

The inhibition rate of tumor growth and the rate of tumor regression were calculated from the average value of the tumor volume according to the formula below. The rate of tumor regression was calculated with respect to a group of the inhibition rate of tumor growth> 100%.

Inhibition rate of tumor growth (%)=(1 - average of tumor volume growth in each group/average of tumor volume growth in solvent group)x100

Rate of tumor regression (%)=(1 - average tumor volume in each group on measuring day/average tumor volume in each group when dividing groups)x100

The anti-tumor effect of Test Compound A1 on the final measuring day is shown in Table 5.

Human AML-derived KG-1 cells can be purchased from, for example, ATCC or the like.

[Table 5]

| Dose | Inhibition or regression rate |
|---|---|
| 2 mg/kg | 54% inhibition |
| 4 mg/kg | 70% inhibition |
| 8 mg/kg | 8% regression |

Test Example 6 Anti-Tumor Test 2 on Tumor-bearing Mice transplanted with Human AML-derived KG-1 Cell

4 to 5 wees old male Balb/c nude mice (CHARLES RIVER LABORATORIES JAPAN, INC.) were injected and implanted, under the skin on the back, with 3 x $10^6$ KG-1 cells suspended to a mixed solution of PBS and Matrigel (registered trademark) in 1:1. Dividing into groups was performed when tumor volume reached 100 to 200 $mm^3$, and administering of the test compounds was started. The test was performed on each of the 5 mice in the solvent group and the compound administered group. By oral administration, 6% cyclodextrin aqueous solution for the solvent group, and 6% cyclodextrin aqueous solution in which the test compound was mixed by the dosage shown in Table 6 for the compound administered group were administered. The administration was performed once a day for 21 days, and the body weight and the tumor diameter were measured twice a week. For calculation of the tumor volume, the formula below was used.

$$[\text{Tumor volume } (mm^3)]=[\text{major axis of tumor (mm)}]\times[\text{minor axis of tumor (mm)}]^2\times 0.5$$

The inhibition rate of tumor growth and the rate of tumor regression were calculated from the average value of the tumor volume according to the formula below. The rate of tumor regression was calculated with respect to a group of the inhibition rate of tumor growth> 100%.

$$\text{Inhibition rate of tumor growth (\%)}=(1-\text{average of tumor volume growth in each group/average of tumor volume growth in solvent group})\times 100$$

$$\text{Rate of tumor regression (\%)}=(1-\text{average tumor volume in each group on measuring day/average tumor volume in each group when dividing groups})\times 100$$

The anti-tumor effect of Test Compounds A1, B1, C1, and D1 on the final measuring day is shown in Table 6.

KG-1 cells were purchased from ATCC.

[Table 6]

| Test Compound | Dosage (mg/kg) | Inhibition rate or regression rate |
|---|---|---|
| A1 | 8 | 99% inhibition |
| B1 | 8 | 31 % regression |
| C1 | 1 | 29% regression |
| D1 | 1 | 95% inhibition |

Test Example 7 Anti-Tumor Test 1 on Tumor-bearing Mice transplanted with Human AML-derived MV-4-11 Cell

4 to 5 week old male NOD/SCID nude mice (CHARLES RIVER LABORATORIES JAPAN, INC.) were injected and implanted, under the skin on the back, with 5 x $10^6$ MV-4-11 cells suspended to a mixed solution of PBS and Matrigel (registered trademark) in 1:1. After 7 days of implantation, administering of the test compounds was started. The test was performed on each of the 5 mice in the solvent group (6% cyclodextrin aqueous solution) and the test compound

administered group (Test Compound A1 was mixed to the solvent group by a dosage of 8 mg/kg). The administration was performed once a day for 19 days by oral administration, and the body weight and the tumor diameter were measured twice a week. For calculation of the tumor volume, the formula below was used.

$$[\text{Tumor volume } (\text{mm}^3)]=[\text{major axis of tumor (mm)}]\times[\text{minor axis of tumor (mm)}]^2\times 0.5$$

The inhibition rate of tumor growth was calculated from the average value of the tumor volume according to the formula below.

$$\text{Inhibition rate of tumor growth } (\%)=(1-\text{average of tumor volume growth in each group/average of tumor volume growth in solvent group})\times 100$$

The anti-tumor effect of Test Compound A1 on the final measuring day is shown in Table 7.

Human AML-derived MV-4-11 cells were purchased from ATCC.

[Table 7]

| Dose | Inhibition rate |
|---|---|
| 8 mg/kg | 96% |

Test Example 8 Anti-Tumor Test 2 on Tumor-bearing Mice transplanted with Human AML-derived MV-4-11 Cell

4 to 5 week old male NOD/SCID nude mice (CHARLES RIVER LABORATORIES JAPAN, INC.) were injected and implanted, under the skin on the back, with 5 x $10^6$ MV-4-11 cells suspended to a mixed solution of PBS and Matrigel (registered trademark) in 1:1. Administering of the test compounds was started when tumor volume reached 100 to 250 $mm^3$. The test was performed on each of the 5 mice in the solvent group and the compound administered group. By oral administration, 6% cyclodextrin aqueous solution for the solvent group, and 6% cyclodextrin aqueous solution in which the test compound was mixed by the dosage shown in Table 8 for the compound administered group were administered. The administration was performed once a day by oral administration, and the body weight and the tumor diameter were measured twice a week. For calculation of the tumor volume, the formula below was used.

$$[\text{Tumor volume } (\text{mm}^3)]=[\text{major axis of tumor (mm)}]\times[\text{minor axis of tumor (mm)}]^2\times 0.5$$

The inhibition rate of tumor growth and the rate of tumor regression were calculated from the average value of the tumor volume according to the formula below. The rate of tumor regression was calculated with respect to a group of the inhibition rate of tumor growth> 100%.

$$\text{Inhibition rate of tumor growth } (\%)=(1-\text{average of tumor volume growth in each group/average of tumor volume growth in solvent group})\times 100$$

$$\text{Rate of tumor regression } (\%)=(1-\text{average tumor volume in each group on measuring day/average tumor volume in each group when dividing groups})\times 100$$

The anti-tumor effect of Test Compounds A1, B1, C1, and D1 on the final measuring day (14 days after starting the administration) is shown in Table 8.

Human AML-derived MV-4-11 cells were purchased from ATCC.

[Table 8]

| Test Compound | Dosage (mg/kg) | Inhibition rate or regression rate |
|---|---|---|
| A1 | 8 | 70% regression |
| B1 | 8 | 76% regression |

(continued)

| Test Compound | Dosage (mg/kg) | Inhibition rate or regression rate |
|---|---|---|
| C1 | 1 | 77% regression |
| D1 | 1 | 90% inhibition |

Test Example 9 Anti-Tumor Test 1 on Mice Tumor-bearing DLBCL-derived Cell

4 to 5 week old male Balb/c nude mice (CHARLES RIVER LABORATORIES JAPAN, INC.) were injected and implanted, under the skin on the back, with 3 x $10^6$ DB cells or WSU-DLCL-2 cells both derived from DLBCL suspended to a mixed solution of PBS and Matrigel (registered trademark) in 1:1. Dividing into groups was performed when the average tumor volume reached 400 to 500 $mm^3$, and administering of the test compounds was started. The test was performed on each of the 5 mice in the solvent group (6% cyclodextrin aqueous solution) and the compound administered group (Test Compound A1 was mixed to the solvent group by a dosage of 8 mg/kg). The administration was performed once a day for 16 days (DB) or 17 days (WSU-DLCL-2) by oral administration, and the body weight and the tumor diameter were measured twice a week. For calculation of the tumor volume, the formula below was used.

$$[\text{Tumor volume } (mm^3)] = [\text{major axis of tumor (mm)}] \times [\text{minor axis of tumor (mm)}]^2 \times 0.5$$

The rate of tumor regression was calculated from the average value of the tumor volume according to the formula below.

$$\text{Rate of tumor regression (\%)} = (1 - \text{average tumor volume in each group on measuring day} / \text{average tumor volume in each group when dividing groups}) \times 100$$

The anti-tumor effect of Test Compound A1 on the final measuring day is shown in Table 9.

DB cells and WSU-DLCL-2 cells derived from DLBCL can be purchased from ATCC, German Collection of Microorganisms and Cultures, or the like.

[Table 9]

| DLBCL cell line | Regression rate |
|---|---|
| DB | 67% |
| WSU-DLCL-2 | 72% |

Test Example 10 Anti-Tumor Test 2 on Mice Tumor-bearing DLBCL-derived Cell

4 to 5 week old male Balb/c nude mice (CHARLES RIVER LABORATORIES JAPAN, INC.) were injected and implanted, under the skin on the back, with 3 x $10^6$ DB cells or SU-DHL-4 cells both derived from DLBCL suspended to a mixed solution of PBS and Matrigel (registered trademark) in 1:1. Dividing into groups was performed when tumor volume reached 100 to 250 $mm^3$, and administering of the test compounds was started. The test was performed on each of the 5 mice in the solvent group and the compound administered group. By oral administration, 6% cyclodextrin aqueous solution for the solvent group, and 6% cyclodextrin aqueous solution in which the test compound was mixed by the dosage shown in Table 10 for the compound administered group were administered. The administration was performed once a day for 21 days by oral administration, and the body weight and the tumor diameter were measured twice a week. For calculation of the tumor volume, the formula below was used.

$$[\text{Tumor volume } (mm^3)] = [\text{major axis of tumor (mm)}] \times [\text{minor axis of tumor (mm)}]^2 \times 0.5$$

The inhibition rate of tumor growth and the rate of tumor regression were calculated from the average value of the tumor volume according to the formula below. The rate of tumor regression was calculated with respect to a group of the inhibition rate of tumor growth> 100%.

Inhibition rate of tumor growth (%)=(1-average of tumor volume growth in each group/average of tumor volume growth in solvent group)x100

Rate of tumor regression (%)=(1-average tumor volume in each group on measuring day/average tumor volume in each group when dividing groups)x100

The anti-tumor effect of Test Compounds A1, B1, C1, and D1 on the final measuring day is shown in Table 10.
DB cells and SU-DHL-4 derived from DLBCL were purchased from ATCC.

[Table 10]

| Test Compound | Dosage (mg/kg) | Inhibition rate or regression rate DB | Inhibition rate SU-DHL-4 |
|---|---|---|---|
| A1 | 8 | 66% regression | 64% |
| B1 | 8 | 92% regression | 87% |
| C1 | 1 | 69% regression | 100% |
| D1 | 1 | 96% inhibition | 40% |

From the above results, it was confirmed that Compound A, Compound B, Compound C, and Compound D which are active ingredients of a pharmaceutical composition of the present invention inhibit Complex I and have the effect of activating AMPK. In addition, it was confirmed that the compounds have an anti-tumor effect with respect to tumor-bearing mice transplanted with colorectal cancer cells, tumor-bearing mice transplanted with DLBCL-derived cells, and tumor-bearing mice transplanted with AML-derived cells.
Accordingly, a compound selected from Compound A, Compound B, Compound C, and Compound D or a pharmaceutically acceptable salt can be used for treating colorectal cancer, particularly PIK3CA mutation-positive colorectal cancer, or PIK3CA mutation-positive and BRAF mutation-positive colorectal cancer, for treating leukemia in which mitochondrial Complex I is involved, particularly AML, and for treating malignant lymphoma in which mitochondrial Complex I is involved, particularly DLBCL.
A pharmaceutical composition comprising a compound selected from Compound A, Compound B, Compound C, and Compound D or a pharmaceutically acceptable salt thereof as an active ingredient may include excipients as an arbitrary additive, or can be prepared by methods which are commonly used, using excipients commonly used in this field, that is, pharmaceutical excipients, pharmaceutical carrier, or the like.
Administration may be any form of oral administration by a tablet, a pill, a capsule, a granule, powder, a liquid, and the like, or parenteral administration by intra-articular, intravenous, intramuscular, and the like injections, a suppository, a transdermal solution, an ointment, a transdermal patch, a transmucosal solution, a transmucosal patch, and the like.
As a solid composition for the oral administration, a tablet, powder, a granule, and the like is used. In such solid composition, one or two or more kinds of active ingredients are mixed with at least one inert excipient. The composition may contain an inert additive, for example, a lubricant, a disintegrant, a stabilizer, a solubilizer, and the like. The tablet or the pill may be coated with a film of sugar or a stomach-soluble, or enteric-soluble substance, if necessary.
A liquid composition for the oral administration includes an emulsion, a solution preparation, a suspension, a syrup or an elixir, and the like which is pharmaceutically acceptable, and includes a generally used inert diluent, for example, purified water or ethanol. The liquid composition may contain adjuvants such as a solubilizing agent, a wetting agent, and a suspension, a sweetener, a flavor, an aromatic, or a preservative in addition to the inert diluent.
The injection for the parenteral administration includes a sterile aqueous or non-aqueous solution preparation, a suspension or an emulsion. As the aqueous solvent, for example, distilled water for injection or physiological saline is included. As the non-aqueous solvent, for example, alcohols such as ethanol are included. Such a composition may further include a tonicity agent, a preservative, a wetting agent, an emulsifier, a dispersant, a stabilizer, or a solubilizer. These are sterilized by for example, filtration through a bacteria-retaining filter, mixing of a germicide, or irradiation. In addition, these can also be used in a manner in which a sterile solid composition is prepared, and is dissolved or suspended in sterile water or a sterile solvent for injection before being used.
As an external application, an ointment, a plaster, a cream, a jelly, a poultice, a spray, a lotion, and the like is included. A generally used ointment base, lotion base, aqueous or non-aqueous solution, suspension, emulsion, and the like is contained.
The transmucosal agent such as a transnasal agent and the like is used in a solid, liquid, or semi-solid form,

and can be prepared according to methods known in the related art. For example, a known excipient, a pH adjuster, a preservative, a surfactant, a lubricant, a stabilizer, a thickener, and the like may be suitably added. In administration, it is possible to use an appropriate device for inhalation or insufflation. For example, by using a known device such as a metered dose inhaler device and the like, or a nebulizer, the administration can be performed as a powder of a compound alone or of a prescribed mixture, or as a solution or a suspension in combination with a carrier which is pharmaceutically acceptable. A dry powder inhaler and the like may be an inhaler for single or multiple administrations, and it is possible to use dry powder or a powder-containing capsule. Alternatively, this may be in a form of a pressurized aerosol spray and the like that uses an appropriate propellant, for example, a suitable gas such as chlorofluoroalkane or carbon dioxide, and the like.

In a case of normal oral administration, a daily dose is approximately 0.001 to 100 mg/kg of body weight, preferably 0.1 to 30 mg/kg, and more preferably 0.1 to 10 mg/kg, and this dose is administered at once or in 2 to 4 divided doses. In a case of an intravenous administration, approximately 0.0001 to 10 mg/kg of body weight is suitable for a daily dose, and this dose is administered at once or in multiple divided doses per day. In addition, as the transmucosal agent, approximately 0.001 to 100 mg/kg of body weight is administered at once or in multiple divided doses per day. The dose is appropriately determined according to individual cases in consideration of symptoms, age, gender, or the like.

The amount differs depending on the type of administration route, dosage form, administration site, excipients, and additives, but the pharmaceutical composition of the present invention contains 0.01 to 100% by weight, and in a certain embodiment, 0.01 to 50% by weight of a compound selected from Compound A, Compound B, Compound C, and Compound D or a pharmaceutically acceptable salt thereof which are active ingredients.

The pharmaceutical composition of the present invention can be used together with various agents for treating diseases which is believed to exhibit effectiveness with respect to colorectal cancer, leukemia, or malignant lymphoma. For use in combination, coadministration or separate administration in succession may be performed, or administration may be performed at a desired time interval. When performing the coadministration, these may be a combination agent, or may be formulated separately.

## Examples

Hereinafter, preparation methods for Compound A, Compound B, Compound C, and Compound D will be described in detail based on examples. In addition, preparation methods for starting compounds thereof will be described in Preparation Examples. In addition, the preparation methods for Compound A, Compound B, Compound C, and Compound D are not limited to the preparation methods in the specific examples shown below, and the compounds can also be prepared by using another combination of the preparation methods, or a method obvious to those skilled in the art.

In the present specification, naming a software such as ACD/Name (registered trademark, manufactured by Advanced Chemistry Development, Inc.) or the like is used in naming of compounds in some cases.

In addition, for the sake of convenience, a concentration mol/1 is expressed by M. For example, a 1 M aqueous sodium hydroxide solution means a 1 mol/1 aqueous sodium hydroxide solution.

The powder X-ray diffraction was measured using RINT-TTRII (manufactured by RIGAKU Corporation) under the conditions of tube: Cu, tube current: 300 mA, tube voltage: 50 kV, sampling width: 0.020°, scanning speed: 4°/min, wavelength: 1.54056 A, and measurement diffraction angle range (2θ): 2.5° to 40°. Handling of a device including a data process was in accordance with the methods and procedures instructed on each device.

Each crystal was characterized by a powder X-ray diffraction pattern, respectively, but judging from the nature of data of the powder X-ray diffraction, the crystal lattice distance and the overall pattern are important in determining the identity of the crystal, and the diffraction angle and diffraction intensity are not to be strictly interpreted since these may vary slightly in accordance with the direction of crystal growth, the particle size, and measurement conditions. The diffraction angle (2θ(°)) of the powder X-ray diffraction is interpreted in consideration of an error range that is generally acceptable in the measuring method, and the error range is ±0.2° in a certain embodiment.

### Preparation Example 1

N-[3-(dimethylamino)propyl]-N'-ethylcarbodiimide hydrochloride (1.2 g) was added to a mixture of 5-bromo-1H-benzimidazol-2-carboxylic acid (1.0 g), 1-[4-(trifluoromethyl)benzyl]piperazine (1.0 g), 1H-benzotriazol-1-ol (840 mg), and N,N-dimethylformamide (10 ml: hereinafter, abbreviated as DMF), followed by stirring at room temperature overnight. A saturated aqueous sodium hydrogen carbonate solution was added to the reaction mixture, followed by stirring at room temperature for 1 hour, and the resulting solid was collected by filtration, followed by drying under reduced pressure. The obtained solid was dissolved in a mixture of chloroform (100 ml) and ethanol (1 ml) while heating to reflux. The mixture was cooled to room temperature and then hexane (100 ml) was added thereto. The resulting solid was collected by filtration, followed by drying under reduced pressure, thereby obtaining (5-bromo-1H-benzimidazol-2-yl){4-[4-(trifluoromethyl)benzyl]piperazin-1-yl}methanone (1.4 g) as a solid.

Preparation Example 2

A mixture of (5-bromo-1H-benzimidazol-2-yl){4-[4-(trifluoromethyl)benzyl]piperazin-1-yl}methanone (1.2 g), tert-butyl 4-(4,4,5,5-tetramethyl-1,3,2-dioxaborolan-2-yl)-3,6-dihydropyridine-1(2H)-carboxylate (1.6 g), tetrakis(triphenylphosphine)palladium (590 mg), sodium carbonate (2.2 g), dioxane (40 ml), and water (10 ml) was stirred at 95°C for 24 hours in an argon atmosphere, and then cooled to room temperature. Water was added to the reaction mixture, and extraction was carried out using ethyl acetate. After the organic layer was dried over anhydrous sodium sulfate, the desiccant was removed, and then the solvent was evaporated under reduced pressure. The obtained residue was purified by silica gel column chromatography (chloroform-methanol), thereby obtaining tert-butyl 4-[2-({4-[4-(trifluoromethyl)benzyl]piperazin-1-yl}carbonyl)-1H-benzimidazol-5-yl]-3,6-dihydropyridine-1(2H)-carboxylate (1.2 g) as an oily material.

Preparation Example 3

10% palladium-activated charcoal (approximately 50% water-containing product, 500 mg) was added to an ethanol (40 ml) solution of tert-butyl 4-[2-({4-[4-(trifluoromethyl)benzyl]piperazin-1-yl} carbonyl)-1 H-benzimidazol-5-yl]-3,6-dihydropyridine-1(2H)-carboxylate (1.4 g), followed by stirring at room temperature for 6 hours in a hydrogen atmosphere. The insoluble material was removed, and then the solvent was evaporated under reduced pressure. 10% palladium-activated charcoal (approximately 50% water-containing product, 500 mg) was added to an ethanol (40 ml) solution of the obtained residue, followed by stirring at room temperature for 4 hours in a hydrogen atmosphere of 3.0 kgf/$cm^2$. The insoluble material was removed, and then the solvent was evaporated under reduced pressure. 20% palladium hydroxide-activated charcoal (approximately 50% water-containing product, 800 mg) was added to a methanol (41 ml) solution of the obtained residue, followed by stirring at room temperature for 24 hours in a hydrogen atmosphere of 3.0 kgf/$cm^2$. The insoluble material was removed, and then the solvent was evaporated under reduced pressure. The obtained residue was purified by silica gel column chromatography (chloroform-methanol), thereby obtaining tert-butyl 4-[2-({4-[4-(trifluoromethyl)benzyl]piperazin-1-yl}carbonyl)-1H-benzimidazol-5-yl]piperidine-1-carboxylate (1.1 g) as an oily material.

Preparation Example 4

A 4 M hydrogen chloride/ethyl acetate solution (5 ml) was added to an ethyl acetate (30 ml) solution of tert-butyl 4-[2-({4-[4-(trifluoromethyl)benzyl]piperazin-1-yl}carbonyl)-1H-benzimidazol-5-yl]piperidine-1-carboxylate (1.1 g), the reaction mixture was stirred at room temperature for 6 hours, and then left to stand overnight. The solvent was evaporated under reduced pressure, and then ethyl acetate and hexane were added to the obtained residue. The resulting solid was collected by filtration, followed by drying under reduced pressure, thereby obtaining hydrochloride (740 mg: a molar ratio to hydrogen chloride was undetermined) of [5-(piperidin-4-yl)-1H-benzimidazol-2-yl]{4-[4-(trifluoromethyl)benzyl]piperazin-1-yl}methanone as a solid.

Preparation Example 5

Trifluoroacetic acid (1 ml) was added to a dichloromethane (2 ml) solution of tert-butyl 4-[2-({4-[4-(trifluoromethyl)benzyl]piperazin-1-yl}carbonyl)-1H-benzimidazol-5-yl]piperidine-1-carboxylate (270 mg), followed by stirring at room temperature for 30 minutes. A saturated aqueous sodium hydrogen carbonate solution was added to the reaction mixture, and extraction was carried out using chloroform. The organic layer was washed with a saturated aqueous sodium chloride solution and then dried over anhydrous sodium sulfate. The desiccant was removed, and then the solvent was evaporated under reduced pressure. The obtained residue was purified by amino silica gel column chromatography (chloroform-methanol), thereby obtaining [5-(piperidin-4-yl)-1H-benzimidazol-2-yl]{4-[4-(trifluoromethyl)benzyl]piperazin-1-yl}methanone (150 mg) as an amorphous material.

Preparation Example 6

A mixture of 6-bromo-1H-indole-2-carboxylate (1.0 g), N-[3-(dimethylamino)propyl]-N'-ethylcarbodiimide hydrochloride (1.1 g), 1H-benzotriazol-1-ol (770 mg), and dichloromethane (15 ml) was stirred at room temperature for 10 minutes. 4-(Piperazin-1-ylmethyl)benzonitrile dihydrochloride (1.2 g) and N,N-diisopropylethylamine (1.6 ml) were added to the reaction mixture, followed by stirring at room temperature overnight. After a saturated aqueous sodium hydrogen carbonate solution was added to the reaction mixture, extraction was carried out using chloroform, and then extraction was carried out using chloroform-methanol. After the organic layer was dried over anhydrous magnesium sulfate, the desiccant was removed, and then the solvent was evaporated under reduced pressure. The obtained residue was purified by silica gel column chromatography (chloroform-methanol), thereby obtaining 4-({4-[(6-bromo-1H-indol-2-yl)carbonyl]piperazin-1-yl}methyl)benzonitrile (1.1 g) as a solid.

Preparation Example 7

A mixture of 4-({4-[(6-bromo-1H-indol-2-yl)carbonyl]piperazin-1-yl}methyl)benzonitrile (1.1 g), tert-butyl 4-(4,4,5,5-tetramethyl-1,3,2-dioxaborolan-2-yl)-3,6-dihydropyridine-1(2H)-carboxylate (1.6 g), tetrakis(triphenylphosphine)palladium (480 mg), sodium carbonate (790 mg), dioxane (18 ml), and water (1.8 ml) was stirred at 100°C overnight in an argon atmosphere, and then cooled to room temperature. Water was added to the reaction mixture, and extraction was carried out using chloroform. After the organic layer was dried over anhydrous sodium sulfate, the desiccant was removed, and then the solvent was evaporated under reduced pressure. The obtained residue was purified by silica gel column chromatography (chloroform-methanol, and then hexane-ethyl acetate), and then the obtained solid was washed with diisopropyl ether, thereby obtaining tert-butyl 4-(2-{[4-(4-cyanobenzyl)piperazin-1-yl]carbonyl}-1H-indol-6-yl)-3,6-dihydropyridine-1(2H)-carboxylate (470 mg) as a solid.

Preparation Example 8

10% palladium-activated charcoal (approximately 50% water-containing product, 230 mg) was added to a mixture of tert-butyl 4-(2-{[4-(4-cyanobenzyl)piperazin-1-yl]carbonyl}-1H-indol-6-yl)-3,6-dihydropyridine-1(2H)-carboxylate (470 mg), tetrahydrofuran (hereinafter, abbreviated as THF) (14 ml), and ethanol (3 ml), followed by stirring at room temperature for 2 hours in a hydrogen atmosphere. After the insoluble material was removed, the solvent was evaporated under reduced pressure, and then 10% palladium-activated charcoal (approximately 50% water-containing product, 230 mg) was added to a mixture of the obtained residue, THF (14 ml), and ethanol (3 ml), followed by stirring at room temperature overnight in a hydrogen atmosphere. After the insoluble material was removed, the solvent was evaporated under reduced pressure. After 20% palladium hydroxide-activated charcoal (approximately 50% water-containing product, 230 mg) was added to a mixture of the obtained residue, THF (14 ml), and ethanol (3 ml), the reaction mixture was stirred at room temperature for 4 hours in a hydrogen atmosphere of 3.0 $kgf/cm^2$, and then left to stand for 3 days. The insoluble material was removed, and then the solvent was evaporated under reduced pressure. The obtained residue was purified by amino silica gel column chromatography (chloroform-methanol), thereby obtaining tert-butyl 4-[2-(piperazin-1-ylcarbonyl)-1H-indol-6-yl]piperidine-1-carboxylate (360 mg) as an oily material.

Preparation Example 9

Sodium triacetoxyborohydride (360 mg) was added to a mixture of tert-butyl 4-[2-(piperazin-1-ylcarbonyl)-1H-indol-6-yl]piperidine-1-carboxylate (350 mg), 4-formylbenzonitrile (140 mg), and dichloromethane (3 ml), followed by stirring at room temperature for 1.5 hours. A saturated aqueous sodium hydrogen carbonate solution and methanol were added to the reaction mixture, and extraction was carried out using chloroform. After the organic layer was dried over anhydrous sodium sulfate, the desiccant was removed, and then the solvent was evaporated under reduced pressure. The obtained residue was purified by silica gel column chromatography (chloroform-methanol), thereby obtaining tert-butyl 4-(2-{[4-(4-cyanobenzyl)piperazin-1-yl]-carbonyl}-1H-indol-6-yl)piperidine-1-carboxylate (450 mg) as an oily material.

Preparation Example 10

Sodium hydride (containing approximately 45% of a liquid paraffin, 40 mg) was added to a DMF (4 ml) solution of tert-butyl 4-(2-{[4-(4-cyanobenzyl)piperazin-1-yl]-carbonyl}-1H-indol-6-yl)piperidine-1-carboxylate (450 mg) under ice-cooling, followed by stirring at room temperature for 1 hour. Methyl iodide (58 μl) was added to the reaction mixture at room temperature, followed by stirring at room temperature for 1 hour. A saturated aqueous ammonium chloride solution and water were added to the reaction mixture, and extraction was carried out using ethyl acetate. The organic layer was washed with water and a saturated aqueous sodium chloride solution in this order, and then dried over anhydrous sodium sulfate. The desiccant was removed, and then the solvent was evaporated under reduced pressure. The obtained residue was purified by silica gel column chromatography (chloroform-methanol), thereby obtaining tert-butyl 4-(2-{[4-(4-cyanobenzyl)piperazin-1-yl]carbonyl}-1-methyl-1H-indol-6-yl)piperidine-1-carboxylate (200 mg) as an oily material.

Preparation Example 11

Trifluoroacetic acid (500 μl) was added to a dichloromethane (1 ml) solution of tert-butyl 4-(2-{[4-(4-cyanobenzyl)piperazin-1-yl]carbonyl}-1-methyl-1H-indol-6-yl)piperidine-1-carboxylate (200 mg) at room temperature, followed by stirring at room temperature for 2 hours. The solvent was evaporated under reduced pressure, a saturated aqueous

sodium hydrogen carbonate solution and water were added to the obtained residue, and then extraction was carried out using chloroform. The organic layer was dried over anhydrous sodium sulfate, the desiccant was removed, and then the solvent was evaporated under reduced pressure. The obtained residue was purified by amino silica gel column chromatography (chloroform-methanol), thereby obtaining 4-[(4-{[1-methyl-6-(piperidin-4-yl)-1H-indol-2-yl]carbonyl}piperazin-1-yl)methyl]benzonitrile (120 mg) as a solid.

Preparation Example 12

Sodium borohydride (2.6 g) was added in portions plural times to a methanol (100 ml) solution of ethyl 5-ethoxypyrazine-2-carboxylate (4.5 g) under ice-cooling, followed by stirring at room temperature for 6 hours. 1 M hydrochloric acid was added to the reaction mixture so that pH became 4, followed by stirring at room temperature for 15 minutes. A 1 M aqueous sodium hydroxide solution was added to the mixture so that pH became 9, and then extraction was carried out using chloroform. After the organic layer was dried over anhydrous sodium sulfate, the desiccant was removed, and the solvent was evaporated under reduced pressure. The obtained residue was purified by silica gel column chromatography (hexane-ethyl acetate), thereby obtaining (5-ethoxypyrazin-2-yl)methanol (2.6 g) as an oily material.

Preparation Example 13

Thionyl chloride (200 μl) was added to a dichloromethane (3 ml) solution of (5-ethoxypyrazin-2-yl)methanol (150 mg) under ice-cooling, followed by stirring at room temperature for 30 minutes. The reaction mixture was concentrated under reduced pressure, thereby obtaining 2-(chloromethyl)-5-ethoxypyrazine (160 mg) as an oily material.

Preparation Example 14

A mixture of ethyl 5-bromo-1H-indole-2-carboxylate (5.2 g), tert-butyl 4-(4,4,5,5-tetramethyl-1,3,2-dioxaborolan-2-yl)-3,6-dihydropyridine-1 (2H)-carboxylate (13 g), tetrakis(triphenylphosphine)palladium (5.3 g), 2 M aqueous sodium carbonate solution (28 ml), and dioxane (110 ml) was stirred at 95°C for 17 hours in an argon atmosphere, and then cooled to room temperature. Water was added to the reaction mixture, and extraction was carried out using chloroform. After the organic layer was dried over anhydrous sodium sulfate, the desiccant was removed, and then the solvent was evaporated under reduced pressure. The obtained residue was purified by silica gel column chromatography (hexane-ethyl acetate, and then chloroform-methanol) and amino silica gel column chromatography (hexane-ethyl acetate). Hexane-diisopropyl ether was added to the obtained solid (6.5 g), and then powderization was carried out. The solid was collected by filtration, followed by drying under reduced pressure, thereby obtaining ethyl 5-[1-(tert-butoxycarbonyl)-1,2,3,6-tetrahydropyridin-4-yl]-1H-indole-2-carboxylate (5.0 g) as a solid.

Preparation Example 15

20% palladium hydroxide-activated charcoal (approximately 50% water-containing product, 1.0 g) was added to a mixture of ethyl 5-[1-(tert-butoxycarbonyl)-1,2,3,6-tetrahydropyridin-4-yl]-1H-indole-2-carboxylate (5.0 g), ethanol (55 ml), and THF (55 ml), followed by stirring at room temperature for 3 hours in a hydrogen atmosphere. The insoluble material was removed, and then the solvent was evaporated under reduced pressure. The obtained residue was purified by amino silica gel column chromatography (hexane-ethyl acetate), thereby obtaining ethyl 5-[1-(tert-butoxycarbonyl)piperidin-4-yl]-1H-indole-2-carboxylate (4.8 g) as a solid.

Preparation Example 16

Dimethyl sulfate (1.8 ml) was added to a mixture of ethyl 5-[1-(tert-butoxycarbonyl)piperidin-4-yl]-1H-indole-2-carboxylate (4.8 g), cesium carbonate (7.0 g), and acetonitrile (70 ml), followed by stirring at 75°C for 2 hours, and then cooled to room temperature. After ethyl acetate was added to the reaction mixture, washing was performed with water and a saturated aqueous sodium chloride solution in this order. After the organic layer was dried over anhydrous sodium sulfate, the desiccant was removed, and then the solvent was evaporated under reduced pressure, thereby obtaining ethyl 5-[1-(tert-butoxycarbonyl)piperidin-4-yl]-1-methyl-1H-indole-2-carboxylate (5.7 g) as an oily material.

Preparation Example 17

AIM aqueous sodium hydroxide solution (23 ml) was added to a mixture of ethyl 5-[1-(tert-butoxycarbonyl)piperidin-4-yl]-1-methyl-1H-indole-2-carboxylate (5.7 g), dioxane (23 ml), and ethanol (23 ml), followed by stirring at 60°C

overnight, and then cooled to room temperature. 1 M hydrochloric acid (23 ml) was added to the reaction mixture under ice-cooling, and extraction was carried out using chloroform. After the organic layer was dried over anhydrous sodium sulfate, the desiccant was removed, and the solvent was evaporated under reduced pressure, thereby obtaining 5-[1-(tert-butoxycarbonyl)piperidin-4-yl]-1-methyl-1H-indole-2-carboxylate (4.8 g) as an amorphous material.

Preparation Example 18

4-(Piperazin-1-ylmethyl)benzonitrile dihydrochloride (4.0 g) and N-[3-(dimethylamino)propyl]-N'-ethylcarbodiimide hydrochloride (3.1 g) were added to a mixture of 5-[1-(tert-butoxycarbonyl)piperidin-4-yl]-1-methyl-1H-indole-2-carboxylate (4.8 g), 1H-benzotriazol-1-ol (1.9 g), N,N-diisopropylethylamine (6.8 ml), and dichloromethane (55 ml), followed by stirring at room temperature for 2 hours. A saturated aqueous sodium hydrogen carbonate solution and water were added to the reaction mixture, and extraction was carried out using chloroform. After the organic layer was dried over anhydrous sodium sulfate, the desiccant was removed, and then the solvent was evaporated under reduced pressure. The obtained residue was purified by silica gel column chromatography (hexane-ethyl acetate), thereby obtaining tert-butyl 4-(2-{[4-(4-cyanobenzyl)piperazin-1-yl]carbonyl}-1-methyl-1H-indol-5-yl)piperidine-1-carboxylate (6.8 g) as an amorphous material.

Preparation Example 19

Trifluoroacetic acid (5 ml) was added to a dichloromethane (10 ml) solution of tert-butyl 4-(2-{[4-(4-cyanobenzyl)piperazin-1-yl]carbonyl}-1-methyl-1H-indol-5-yl)piperidine-1-carboxylate (6.8 g) at room temperature, followed by stirring at room temperature for 2 hours. The solvent was evaporated under reduced pressure, a saturated aqueous sodium hydrogen carbonate solution was added to the obtained residue, and then extraction was carried out using chloroform. After the organic layer was dried over anhydrous sodium sulfate, the desiccant was removed, and then the solvent was evaporated under reduced pressure, thereby obtaining 4-[(4-{[1-methyl-5-(piperidin-4-yl)-1H-indol-2-yl]carbonyl}piperazin-1-yl)methyl]benzonitrile (7.1 g) as an amorphous material.

Example 1

A mixture of hydrochloride (200 mg) of [5-(piperidin-4-yl)-1H-benzimidazol-2-yl]{4-[4-(trifluoromethyl)benzyl]piperazin-1-yl}methanone, 6-methoxynicotinaldehyde (100 mg), triethylamine (140 μl), acetic acid (100 μl), and dichloromethane (4 ml) was stirred at room temperature for 10 minutes. After sodium triacetoxyborohydride (580 mg) was added to the reaction mixture at room temperature, the reaction mixture was stirred at room temperature for 2 hours, and then left to stand at room temperature overnight. A saturated aqueous sodium hydrogen carbonate solution was added to the reaction mixture, and extraction was carried out using chloroform. After the organic layer was dried over anhydrous sodium sulfate, the desiccant was removed, and then the solvent was evaporated under reduced pressure. After the obtained crude product was purified by amino silica gel column chromatography (chloroform-methanol), tosic acid monohydrate (69 mg) was added to an acetone solution of the obtained oily material (Compound A, 110 mg), and then the solvent was evaporated under reduced pressure. Ethanol (3 ml) and diisopropyl ether (20 ml) were added to the obtained residue, followed by stirring at room temperature. The resulting solid was collected by filtration, and then dried under reduced pressure, thereby obtaining ditosylate salts (180 mg) of 5-{1-[(6-methoxypyridin-3-yl)methyl]piperidin-4-yl}-1H-benzimidazol-2-yl){4-[4-(trifluoromethyl)benzyl]piperazin-1-yl}methanone (Compound A) as an amorphous material. In addition, after stirring a mixture of Compound A (200 mg) prepared in the same manner as above and acetonitrile (10 ml) at 95°C for 30 minutes, tosic acid monohydrate (130 mg) was added thereto. The mixture was cooled to room temperature while stirring, and then stirred at room temperature for 7 days. The resulting solid was collected by filtration, and then dried under reduced pressure, thereby obtaining ditosylate salts (300 mg) of (5-{1-[(6-methoxypyridin-3-yl)methyl]piperidin-4-yl}-1 H-benzimidazol-2-yl) {4-[4-(trifluoromethyl)benzyl]piperazin-1-yl}methanone (Compound A) as a crystal. The powder X-ray diffraction data of this crystal is shown in Table 16 described below.

Example 2

2-(Chloromethyl)-5-methoxypyrazine (16 mg) was added to a mixture of [5-(piperidin-4-yl)-1H-benzimidazol-2-yl]{4-[4-(trifluoromethyl)benzyl]piperazin-1-yl}methanone (47 mg), N,N-diisopropylethylamine (68 μl), acetonitrile (1 ml), and DMF (1 ml), followed by stirring at room temperature for 5 days. Water was added to the reaction mixture, and extraction was carried out using ethyl acetate. After the organic layer was dried over anhydrous sodium sulfate, the desiccant was removed, and then the solvent was evaporated under reduced pressure. After the obtained crude product was purified by silica gel column chromatography (chloroform-methanol), tosic acid monohydrate (24 mg) and ethyl acetate (3 ml) were added to an acetone (2 ml) solution of the obtained oily material (Compound B, 38 mg), followed by

stirring at room temperature overnight. The resulting solid was collected by filtration, and then dried under reduced pressure, thereby obtaining ditosylate salts (53 mg) of (5-{1-[(5-methoxypyrazin-2-yl)methyl]piperidin-4-yl}-1H-benzimidazol-2-yl){4-[4-(trifluoromethyl)benzyl]piperazin-1-yl}methanone (Compound B) as a solid. In addition, after stirring a mixture of Compound B (200 mg) prepared in the same manner as above, acetone (18 ml), and acetonitrile (3 ml) at 80°C, tosic acid monohydrate (130 mg) was added thereto. The mixture was cooled to room temperature while stirring, and then stirred at room temperature for 72 hours. The resulting solid was collected by filtration, and then dried under reduced pressure, thereby obtaining ditosylate salts (300 mg) of (5-{1-[(5-methoxypyrazin-2-yl)methyl]piperidin-4-yl}-1H-benzimidazol-2-yl){4-[4-(trifluoromethyl)benzyl]piperazin-1-yl}methanone (Compound B) as a crystal. The powder X-ray diffraction data of this crystal is shown in Table 16 described below.

Example 3

An acetonitrile (500 μl) solution of 2-(chloromethyl)-5-ethoxypyrazine (53 mg) was added to a mixture of 4-[(4-{[1-methyl-6-(piperidin-4-yl)-1H-indol-2-yl]carbonyl}piperazin-1-yl)methyl]benzonitrile (120 mg), N,N-diisopropylethylamine (160 μl), and acetonitrile (1 ml), followed by stirring at room temperature overnight. After the solvent of the reaction mixture was evaporated under reduced pressure, the obtained residue was purified by silica gel column chromatography (chloroform-methanol) and DIOL silica gel column chromatography (hexane-ethyl acetate). After stirring a mixture of the obtained solid (110 mg) and acetone (3 ml) at 80°C, tosic acid monohydrate (68 mg) was added thereto. The mixture was cooled to room temperature while stirring, and then stirred at room temperature overnight. The resulting solid was collected by filtration, and then dried under reduced pressure, thereby obtaining ditosylate salts (130 mg) of 4-({4-[(6-{1-[(5-ethoxypyrazin-2-yl)methyl]piperidin-4-yl}-1-methyl-1H-indol-2-yl)carbonyl]piperazin-1-yl}methyl)benzonitril (Compound C) as a crystal. The powder X-ray diffraction data of this crystal is shown in Table 16 described below.

Example 4

A dichloromethane (5 ml) solution of 2-(chloromethyl)-5-methoxypyrazine (760 mg) was added to a mixture of 4-[(4-{[1-methyl-5-(piperidin-4-yl)-1H-indol-2-yl]carbonyl}piperazin-1-yl)methyl]benzonitrile (2.0 g), N,N-diisopropylethylamine (2.7 ml), and acetonitrile (10 ml), followed by stirring at room temperature for 5 days. After the solvent of the reaction mixture was evaporated under reduced pressure, the obtained residue was purified by silica gel column chromatography (chloroform-methanol) and amino silica gel column chromatography (hexane-ethyl acetate, and then chloroform-methanol), thereby obtaining amorphous material (1.4 g). After the obtained amorphous material (1.2 g) was purified by silica gel column chromatography (chloroform-ethyl acetate), a mixture of the obtained solid (760 mg) and acetone (100 ml) was heated and stirred at 80°C for 30 minutes, and then tosic acid monohydrate (510 mg) was added thereto. The mixture was cooled to room temperature while stirring, and then stirred at room temperature for 4 hours. The resulting solid was collected by filtration, and then dried under reduced pressure, thereby obtaining ditosylate salts (1.1 g) of 4-({4-[(5-{1-[(5-methoxypyrazin-2-yl)methyl]piperidin-4-yl}-1-methyl-1H-indol-2-yl)carbonyl]piperazin-1-yl}methyl)benzonitrile (Compound D) as a crystal. The powder X-ray diffraction data of this crystal is shown in Table 16 described below.

The structure and physicochemical data of the compounds of the preparation examples and the compounds of examples are shown in Tables 11 to 16 described below.

The following abbreviations may be used in Tables 11 to 16 described below. Pre: preparation example No, Ex: example No, Str: chemical structure formula, Dat: physicochemical data, ESI+: m/z value in mass spectrometry (ionization method ESI, $[M+H]^+$ unless otherwise specified), ESI-: m/z value in mass spectrometry (ionization method ESI, $[M-H]^-$ unless otherwise specified), APCI/ESI: APCI/ESI-MS (atmospheric pressure chemical ionization method APCI, APCI/ESI refers to the simultaneous measurement of APCI and ESI, and APCI/ESI+ is $[M+H]^+$), NMR1: δ (ppm) of peak in $^1$H-NMR in $CD_3OD$, NMR2: δ (ppm) of peak in $^1$H-NMR in DMSO-$d_6$, Me: methyl, Et: ethyl, Boc: tert-butoxycarbonyl, and 2θ(°): diffraction angle of powder X-ray diffraction.

Furthermore, in the chemical structure formulas, xHCl indicates that the compound is a hydrochloride, but the molar ratio to hydrogen chloride is undetermined, and 2TsOH indicates that the compound is a ditosylate salt, respectively.

[Table 11]

| Pre | Str | Dat |
|---|---|---|
| 1 | 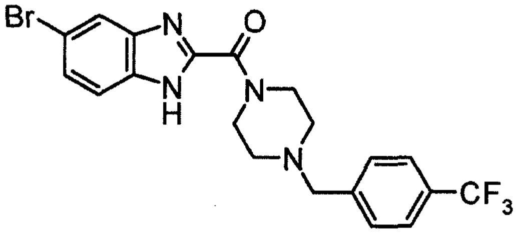 | ESI+: 467, 469 |
| 2 | 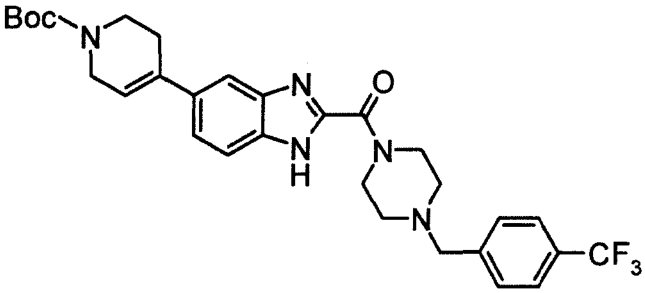 | ESI+: 570 |
| 3 | 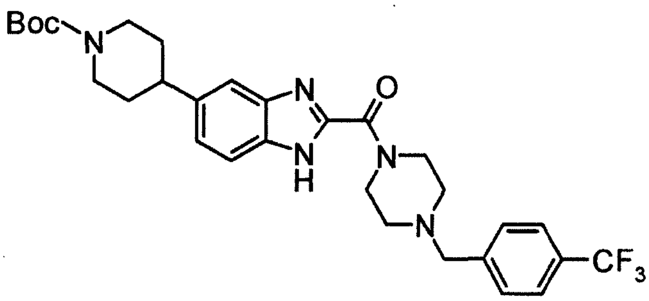 | ESI+: 572 |
| 4 | 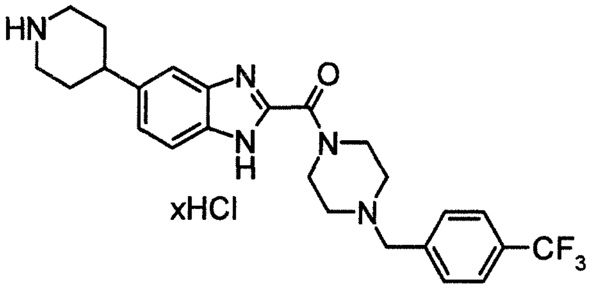 | ESI+: 472 |
| 5 | 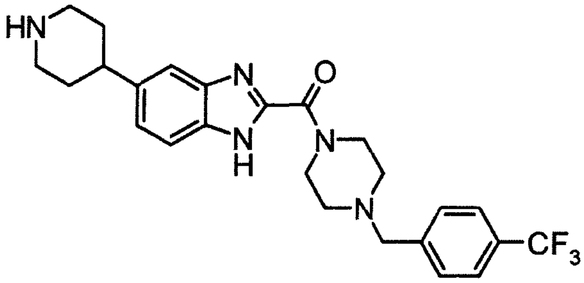 | APCI/ESI+: 472 |

[Table 12]

| Pre | Str | Dat |
|---|---|---|
| 6 | 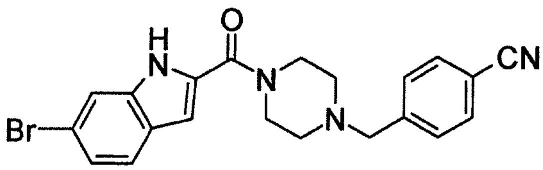 | ESI+: 423, 425 |
| 7 | 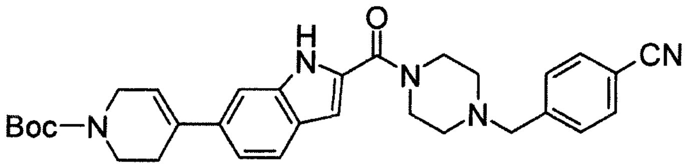 | ESI+: 526 |
| 8 | 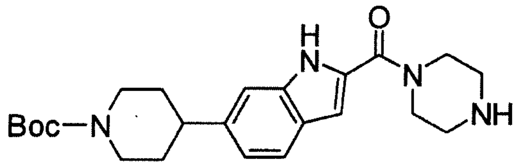 | ESI-: 411 |
| 9 | 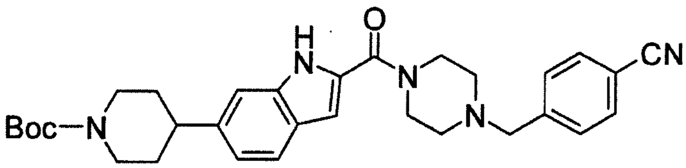 | ESI-: 526 |
| 10 | 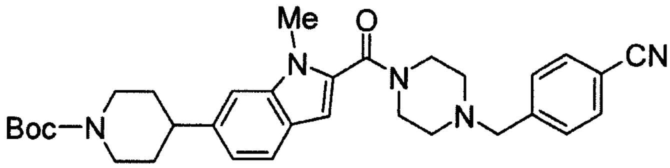 | ESI+: 542 |
| 11 | 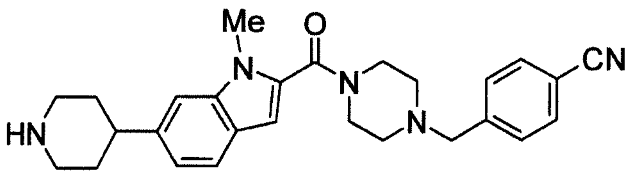 | ESI+: 442 |
| 12 | 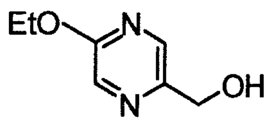 | ESI+: 155 |

[Table 13]

| Pre | Str | Dat |
|---|---|---|
| 13 | 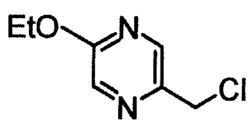 | ESI+: 173, 175 |

(continued)

| Pre | Str | Dat |
|---|---|---|
| 14 | 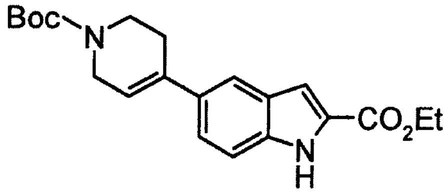 | ESI-: 369 |
| 15 | 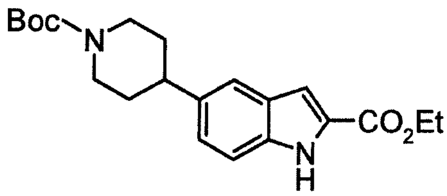 | ESI+: 373 |
| 16 | 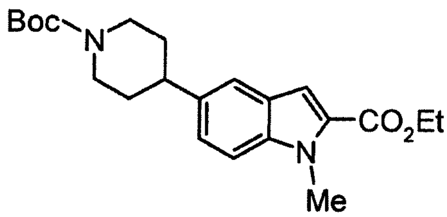 | ESI+: 409 [M+Na]+ |
| 17 | 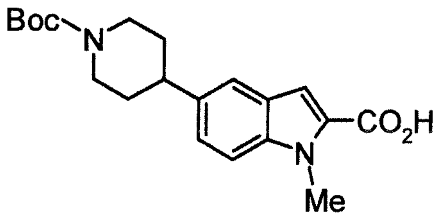 | ESI-: 357 |

[Table 14]

| Pre | Str | Dat |
|---|---|---|
| 18 | 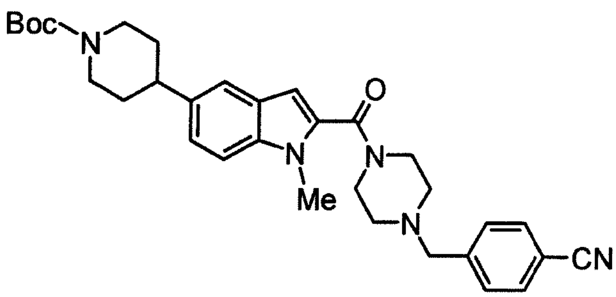 | ESI+: 542 |
| 19 | 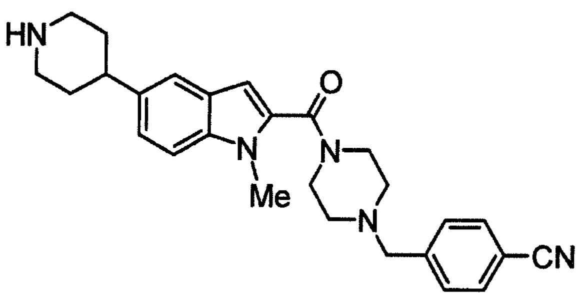 | ESI+: 442 |

[Table 15]

| Ex | Str |
|---|---|
| 1 | 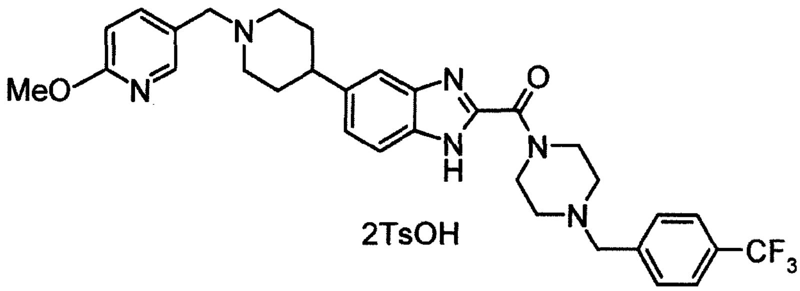 |
| 2 | 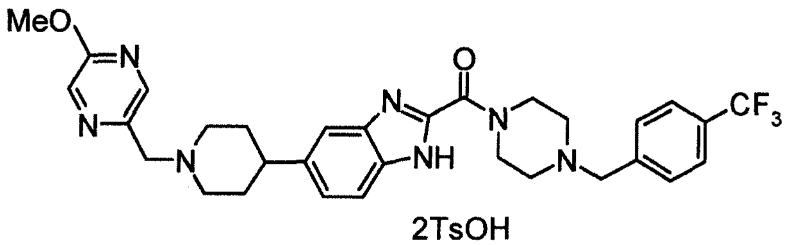 |
| 3 | 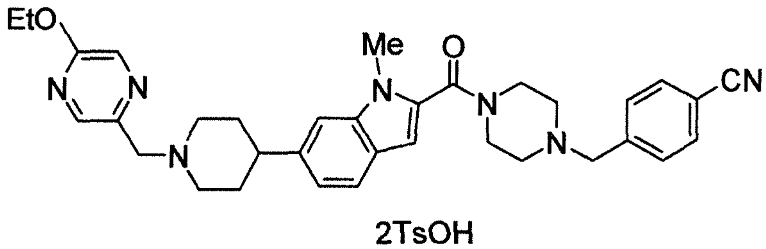 |
| 4 | 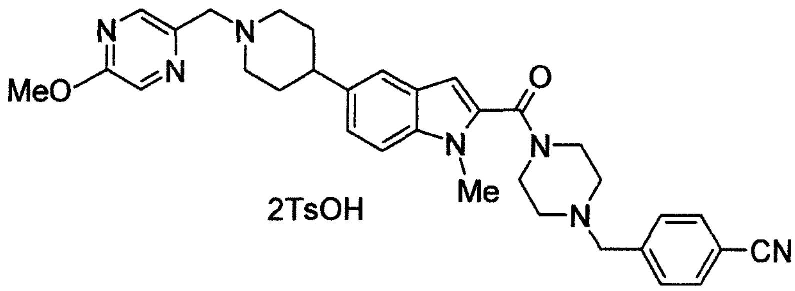 |

[Table 16]

| Ex | Dat |
|---|---|
| 1 | ESI+: 593;<br>NMR1: 1.98-2.21 (4H, m), 2.38 (6H, s), 2.99-3.09 (1H, m), 3.12-3.27 (2H, m), 3.27-3.58 (8H, m), 3.59-3.68 (2H, m), 3.95 (3H, s), 4.36 (2H, s), 4.39-4.51 (2H, m), 6.86-6.92 (1H, m), 7.19-7.24 (4H, m), 7.25-7.30 (1H, m), 7.54-7.65 (2H, m), 7.68-7.83 (8H, m), 7.85 (1H, dd, J = 2.8, 8.4 Hz), 8.31 (1H, d, J = 2.0 Hz);<br>2θ (°) = 6.5, 10.1, 15.2, 16.2, 18.6, 19.6, 20.1, 20.8, 23.3, 25.8 |
| 2 | ESI+: 594;<br>NMR1: 2.01-2.22 (4H, m), 2.35 (6H, s), 3.01-3.11 (1H, m), 3.20-3.80 (12H, m), 4.02 (3H, s), 4.47 (2H, s), 4.52 (2H, s), 7.18-7.25 (4H, m), 7.31 (1H, dd, J = 1.6, 8.6 Hz), 7.55-7.59 (1H, m), 7.64 (1H, d, J = 8.5 Hz), 7.67-7.73 (4H, m), 7.75-7.78 (2H, m), 7.78-7.84 (2H, m), 8.31-8.35 (2H, m);<br>2θ (°) = 6.2, 6.7, 13.3, 15.2, 16.4, 19.0, 20.5, 20.9, 22.6, 24.8 |

(continued)

| Ex | Dat |
|---|---|
| 3 | ESI+: 578<br>NMR2: 1.38 (3H, t, J = 7.0 Hz), 1.89-2.10 (4H, m), 2.29 (6H, s), 2.88-3.00 (1H, m), 3.02-3.69 (12H, m), 3.76 (3H, s), 4.26-4.63 (4H, m), 4.41 (2H, q, J = 7.1 Hz), 6.61-6.79 (1H, m), 6.96-7.03 (1H, m), 7.06-7.14 (4H, m), 7.32 (1H, s), 7.44-7.51 (4H, m), 7.57 (1H, d, J = 8.3 Hz), 7.62-7.81 (2H, m), 7.85-8.10 (2H, m), 8.39 (1H, d, J = 1.2 Hz), 8.42 (1H, d, J = 1.2 Hz), 9.67-9.81 (1H, m), 9.89-10.16 (1H, m);<br>2θ (°) = 3.6, 7.2, 10.9, 16.1, 16.7, 17.2, 19.2, 20.9, 22.8, 26.6 |
| 4 | ESI+: 564<br>NMR2: 1.84-2.07 (4H, m), 2.29 (6H, s), 2.82-2.94 (1H, m), 3.08-3.68 (12H, m), 3.75 (3H, s), 3.97 (3H, s), 4.21-4.66 (4H, m), 6.57-6.80 (1H, m), 7.06-7.13 (4H, m), 7.12-7.19 (1H, m), 7.37-7.43 (1H, m), 7.44-7.54 (5H, m), 7.58-7.79 (2H, m), 7.84-8.10 (2H, m), 8.41 (1H, d, J = 1.2Hz), 8.46 (1H, d, J = 1.3Hz), 9.64-9.83 (1H, m), 9.87-10.16 (1H, m);<br>2θ (°) = 7.5, 9.8, 13.2, 14.7, 15.6, 16.9, 18.8, 19.5, 20.0, 22.6 |

Industrial Applicability

A compound selected from Compound A, Compound B, Compound C, and Compound D or a pharmaceutically acceptable salt thereof, which are active ingredients of a pharmaceutical composition of the present invention, has the effect of inhibiting mitochondrial Complex I and the effect of activating AMPK, and can be used as an active ingredient of a pharmaceutical composition for treating colorectal cancer, particularly PIK3CA mutation-positive colorectal cancer, or PIK3CA mutation-positive and BRAF mutation-positive colorectal cancer, for treating leukemia, particularly AML, and/or for treating malignant lymphoma, particularly DLBCL.

## Claims

**1.** A pharmaceutical composition for treating cancer selected from colorectal cancer, leukemia, and malignant lymphoma, the composition comprising:

a compound selected from (5-{1-[(6-methoxypyridin-3-yl)methyl]piperidin-4-yl}-1H-benzimidazol-2-yl){4-[4-(trifluoromethyl)benzyl]piperazin-1-yl}methanone, (5-{1-[(5-methoxypyrazin-2-yl)methyl]piperidin-4-yl}-1H-benzimidazol-2-yl){4-[4-(trifluoromethyl)benzyl]piperazin-1-yl}methanone, 4-({4-[(6-{1-[(5-ethoxypyrazin-2-yl)methyl]piperidin-4-yl}-1-methyl-1H-indol-2-yl)carbonyl]piperazin-1-yl}methyl)benzonitrile, and 4-({4-[(5-{1-[(5-methoxypyrazin-2-yl)methyl]piperidin-4-yl}-1-methyl-1H-indol-2-yl)carbonyl]piperazin-1-yl}methyl)benzonitrile, or a pharmaceutically acceptable salt thereof as an active ingredient.

**2.** The pharmaceutical composition according to claim 1,
wherein cancer selected from colorectal cancer, leukemia, and malignant lymphoma is colorectal cancer.

**3.** The pharmaceutical composition according to claim 1,
wherein cancer selected from colorectal cancer, leukemia, and malignant lymphoma is leukemia.

**4.** The pharmaceutical composition according to claim 1,
wherein cancer selected from colorectal cancer, leukemia, and malignant lymphoma is malignant lymphoma.

**5.** The pharmaceutical composition according to claim 2,
wherein colorectal cancer is PIK3CA mutation-positive colorectal cancer.

**6.** The pharmaceutical composition according to claim 2,
wherein colorectal cancer is PIK3CA mutation-positive or BRAF mutation-positive colorectal cancer.

**7.** The pharmaceutical composition according to claim 3,
wherein leukemia is acute myeloid leukemia.

**8.** The pharmaceutical composition according to claim 3, wherein leukemia is acute myeloid leukemia in which the PI3K/Akt/mTOR pathway is enhanced.

**9.** The pharmaceutical composition according to claim 4, wherein malignant lymphoma is diffuse large B-cell lymphoma.

**10.** The pharmaceutical composition according to any one of claims 5 to 9, comprising:

(5- {1-[(6-methoxypyridin-3-yl)methyl]piperidin-4-yl}-1H-benzimidazol-2-yl) {4-[4-(trifluoromethyl)benzyl]piperazin-1-yl}methanone, or a pharmaceutically acceptable salt thereof as an active ingredient.

**11.** The pharmaceutical composition according to any one of claims 5 to 9, comprising:

(5-{1-[(5-methoxypyrazin-2-yl)methyl]piperidin-4-yl}-1H-benzimidazol-2-yl){4-[4-(trifluoromethyl)benzyl]piperazin-1-yl}methanone, or a pharmaceutically acceptable salt thereof as an active ingredient.

**12.** The pharmaceutical composition according to any one of claims 5 to 9, comprising:

4-({4-[(6-{1-[(5-ethoxypyrazin-2-yl)methyl]piperidin-4-yl}-1-methyl-1H-indol-2-yl)carbonyl]piperazin-1-yl}methyl)benzonitrile, or a pharmaceutically acceptable salt thereof as an active ingredient.

**13.** The pharmaceutical composition according to any one of claims 5 to 9, comprising:

4-({4-[(5-{1-[(5-methoxypyrazin-2-yl)methyl]piperidin-4-yl}-1-methyl-1H-indol-2-yl)carbonyl]piperazin-1-yl}methyl)benzonitrile, or a pharmaceutically acceptable salt thereof as an active ingredient.

**14.** The pharmaceutical composition according to any one of claims 5 to 9, comprising:

(5-{1-[(6-methoxypyridin-3-yl)methyl]piperidin-4-yl}-1H-benzimidazol-2-yl){4-[4-(trifluoromethyl)benzyl]piperazin-1-yl}methanone ditosylate as an active ingredient.

**15.** The pharmaceutical composition according to any one of claims 5 to 9, comprising:

(5-{1-[(5-methoxypyrazin-2-yl)methyl]piperidin-4-yl}-1H-benzimidazol-2-yl){4-[4-(trifluoromethyl)benzyl]piperazin-1-yl}methanone ditosylate as an active ingredient.

**16.** The pharmaceutical composition according to any one of claims 5 to 9, comprising:

4-({4-[(6-{1-[(5-ethoxypyrazin-2-yl)methyl]piperidin-4-yl}-1-methyl-1H-indol-2-yl)carbonyl]piperazin-1-yl}methyl)benzonitrile ditosylate as an active ingredient.

**17.** The pharmaceutical composition according to any one of claims 5 to 9, comprising:

4-({4-[(5-{1-[(5-methoxypyrazin-2-yl)methyl]piperidin-4-yl}-1-methyl-1H-indol-2-yl)carbonyl]piperazin-l-yl}methyl)benzonitrile ditosylate as an active ingredient.

## INTERNATIONAL SEARCH REPORT

International application No.
PCT/JP2015/084456

A. CLASSIFICATION OF SUBJECT MATTER
*A61K31/497*(2006.01)i, *A61K31/496*(2006.01)i, *A61P35/00*(2006.01)i, *A61P43/00*(2006.01)i, *C07D401/14*(2006.01)n

According to International Patent Classification (IPC) or to both national classification and IPC

B. FIELDS SEARCHED

Minimum documentation searched (classification system followed by classification symbols)
A61K31/497, A61K31/496, A61P35/00, A61P43/00, C07D401/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 02/20008 A1 (THE SCRIPPS RESEARCH INSTITUTE), 14 March 2002 (14.03.2002), page 13, lines 8 to 23; page 14, lines 5 to 12; fig. 15A, 15B, 17 & US 2004/0034239 A1 & AU 8889901 A | 1-17 |
| P,A | WO 2014/199933 A1 (Astellas Pharma Inc.), 18 December 2014 (18.12.2014), claims 8 to 15; test examples 1 to 4; examples 1, 7, 66, 79 & US 2015/0266869 A1 & CA 2914982 A & TW 201536771 A | 1-17 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 March 2016 (04.03.16) | 15 March 2016 (15.03.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0220008 A
- WO 2009132136 A
- WO 2012016217 A
- WO 2014199933 A


### Non-patent literature cited in the description


- *Jpn. J. Clin. Oncol.,* 2013, vol. 43, 685-694
- *J. Clin. Oncol.,* 2004, vol. 22, 229-237
- *Nature,* 2005, vol. 436, 792
- *Oncologist.,* 2011, vol. 16, 404-414
- *Nature,* 2002, vol. 417, 949-954
- *Cell,* 2005, vol. 121, 179-193
- *Hematology Am. Soc. Hematol. Educ. Program,* 2005, 143
- Leukemia treatment manual. 2009, 27
- *Cancer Lett.,* 2014, vol. 346, 188-196
- *Blood.,* 2015, vol. 125, 2120-2130
- *Blood.,* 1997, vol. 89, 3909-3918
- *Blood.,* 2005, vol. 105, 1851-1861
- *Cancer Res.,* 2006, vol. 66, 10269-10273
- *Cancer Res.,* 2007, vol. 15, 6745-6752
- *Blood,* 2010, vol. 116, 4262-4273
- *Annu. Rev. Biochem.,* 1998, vol. 67, 821-855
- *Diabetes Metab.,* 2003, vol. 29 (4), 6S88-94
- *Genes Cells.,* 2003, vol. 8, 65-79
- *Cancer Res.,* 2007, vol. 67, 10804-10812
- *Molecular Cell.,* 2009, vol. 33, 237-247